# EUROPEAN PATENT APPLICATION

(11) **EP 4 502 171 A1**
(43) Date of publication of application: **05.02.2025**
(21) Application number: 23780147.7
(22) Date of filing: 24.03.2023
(51) Int. Cl.: C12Q 1/02, C12M 1/00

(54) **METHOD, DEVICE, AND PROGRAM FOR SEARCHING MEDIUM COMPOSITION AND ESTIMATING CELL CHARACTERISTICS**

(30) Priority: 30.03.2022 JP 2022056627
(71) Applicant: FUJIFILM Corporation, Tokyo 106-8620 (JP)
(72) Inventor: NAGASE, Masaya, Ashigarakami-gun, Kanagawa 258-8577 (JP); TAKEMORI, Sho, Ashigarakami-gun, Kanagawa 258-8577 (JP)
(74) Representative: Klunker IP Patentanwälte PartG mbB
(86) International application number: PCT/JP2023/011773
(87) International publication number: WO 2023/190137

(57) **Abstract**

An aspect of the present invention provides a method, an apparatus, and a program for searching for a culture medium composition and estimating a cell characteristic to allow efficient optimization of a culture result even for a cell whose characteristics are unknown. In a method according to an aspect of the present invention, a processor makes a prediction of, for a target cell, a culture result for a given culture medium composition by using a cell characteristic or an estimated value of a cell characteristic of the target cell; acquires a culture result of the target cell for all or part of the given culture medium composition; performs an estimation of a cell characteristic of the target cell by using the culture medium composition and the acquired culture result or the predicted culture result; performs an evaluation of necessity to further estimate a cell characteristic of the target cell from the acquired culture result; and performs a search and determination of a candidate culture medium composition, based on the necessity and the prediction, the candidate culture medium composition being a culture medium composition for which the culture result is to be acquired next. The processor repeats the prediction of a culture result, the acquisition of a culture result, the estimation of a cell characteristic, the evaluation of the necessity, and the search and determination of a candidate culture medium composition until a determined termination criterion is satisfied.

## Description

### BACKGROUND OF THE INVENTION

### 1. Field of the Invention

The present invention relates to a method, an apparatus, and a program for searching for a culture medium composition and estimating a cell characteristic, and more specifically to a method, an apparatus, and a program for searching for a culture medium composition particularly suitable for a specific cell line.

### 2. Description of the Related Art

Recently, with the spread of gene editing technology and so on, diversification of various cells such as CHO cells (Chinese Hamster Ovary cells) for producing antibody drugs, HEK cells (Human Embryonic Kidney cells) for producing viruses for gene therapy, and iPS cells (Induced Pluripotent Stem cells) for regenerative medicine has progressed. In general, in many cases, culture conditions of cells are optimized to increase the proliferative property of the cells or the yields of various products, and in particular, in many cases, it is desirable to optimize a culture medium for a specific cell. In this case, a typical solution is to change, for example, the content of one or more components little by little on the basis of any known culture medium composition and actually culture cells in a large number of culture media to maximize target productivity such as the proliferative property.

For example, JP2014-503220A describes that a functional enviromics map representing the intensity of activation or repression of elementary cellular functions is constructed and that the functional enviromics map is used to develop an optimized cell culture medium formulation.

### SUMMARY OF THE INVENTION

To optimize a culture medium for a specific cell (unique cell), it is considered to be effective to (1) grasp the characteristics of an unknown cell and (2) identify a culture medium composition in accordance with the characteristics. However, it may be desirable to search for an optimum culture medium for a cell whose characteristics are not necessarily known. This is because it takes cost in terms of money or time to grasp the characteristics of a cell and it may be difficult to perform analysis for grasping the characteristics of a cell due to industrial constraints. Accordingly, there are needs to search for and propose an optimum culture medium composition for a cell with unknown characteristics within a limited range of information.

In general, in the optimization problem described in (2) above, optimization is performed by gradually changing a specific favorable culture medium composition. In (1) described above, in contrast, it is typical to examine responses to various culture medium compositions to clarify cell characteristics, and there is a problem in that a search for an optimum culture medium composition for a cell with unknown characteristics may eventually require time and cost equivalent to those of the typical solution described in the previous section.

As described above, it has been difficult for the conventional techniques to achieve both (1) and (2) described above in a limited development period or a limited number of experiments.

The present invention has been made in view of such circumstances, and an object thereof is to provide a method, an apparatus, and a program for searching for a culture medium composition and estimating a cell characteristic to allow efficient optimization of a culture result even for a cell whose characteristics are unknown.

To achieve the object described above, a method according to a first aspect of the present invention is a method for searching for a culture medium composition for a cell and estimating a cell characteristic. The method includes, by a processor, a prediction step of making a prediction of, for a target cell, a culture result for a given culture medium composition by using a cell characteristic or an estimated value of a cell characteristic of the target cell; an acquisition step of acquiring a culture result of the target cell for all or part of the given culture medium composition; an estimation step of performing an estimation of a cell characteristic of the target cell by using the culture medium composition and the acquired culture result or the predicted culture result; an evaluation step of performing an evaluation of necessity to further estimate a cell characteristic of the target cell from the acquired culture result; a search determination step of performing a search and determination of a candidate culture medium composition, based on the necessity and the prediction step, the candidate culture medium composition being a culture medium composition for which the culture result is to be acquired next in the acquisition step; and a control step of performing a repetition of the prediction step, the acquisition step, the estimation step, the evaluation step, and the search determination step until a determined termination criterion is satisfied.

According to the first aspect, a culture result for a given culture medium composition is predicted by using a cell characteristic or an estimated value of a cell characteristic of a target cell, and a culture result of the target cell for all or part of the given culture medium composition is acquired. A cell characteristic is estimated by using the culture medium composition and the acquired culture result or the predicted culture result. Further, necessity to further estimate a cell characteristic is evaluated from the acquired culture result, a candidate culture medium composition for which the culture result is to be acquired next is searched for and determined based on the necessity and the prediction of the culture result, and the steps described above are repeated until a determined termination criterion is satisfied. As described above, repeating the prediction and acquisition of a culture result, the estimation of a cell characteristic, the evaluation of the necessity to additionally estimate a cell characteristic, and the search and determination of a candidate culture medium composition makes it possible to efficiently optimize a culture result even for a cell whose characteristics are unknown.

In the first aspect and the following aspects, the term "given culture medium composition" means a "specific culture medium composition" or "any culture medium composition", and the amounts of the components of each composition are clearly defined. The term "given culture medium composition" is not limited to being "known" in the sense of being commonly used or having already been prepared, and may be a "new" culture medium to be prepared, that is, a culture medium that has not yet been prepared. However, an "unknown" culture medium with unclear amounts of components is not included in the "given culture medium composition".

In the acquisition step, for example, a result of actual culture performed in a culture evaluation system (an experimental system) may be acquired, or a culture result obtained by simulation or the like without actual culture may be acquired.

A method according to a second aspect is the method according to the first aspect, in which in the evaluation step, the processor performs the evaluation on an assumption that a change in culture result in response to a change in culture medium composition has continuity. In the second aspect, the evaluation is performed on the assumption that "the cell culture result does not greatly vary with minor changes in the composition of the culture medium".

A method according to a third aspect is the method according to the first or second aspect, in which in the estimation step, the processor performs the estimation on an assumption that the cell characteristic of the target cell is similar to a cell characteristic of one or more cells in a known cell group. In the third aspect, the estimation is performed by using cell characteristics of a known cell group as "prior knowledge".

A method according to a fourth aspect is the method according to any one of the first to third aspects, in which when the culture result is regarded as an observation history, a means for the prediction in the prediction step is regarded as an unknown function, and a shape of the unknown function is regarded as the cell characteristic, the processor uses a solution to a bandit problem for estimating an unknown function that best fits the observation history to perform the prediction step, the estimation step, the acquisition step, the evaluation step, and the search determination step. The bandit problem is a problem of optimizing an unknown function with a limited number of trials on the basis of an observation history, and in the fourth aspect, specific components are assigns to the bandit problem.

A method according to a fifth aspect is the method according to the fourth aspect, in which the processor uses a cell simulation including a metabolic pathway model to express the means for the prediction in the prediction step, the unknown function, and/or a similar function for the unknown function.

A method according to a sixth aspect is the method according to the fifth aspect, in which the processor receives an input of culture data of the target cell, determines parameters of the metabolic pathway model from the culture data, and estimates the cell characteristic based on the determined parameters. The sixth aspect specifically defines an aspect of estimation of the cell characteristic. The parameters of the metabolic pathway model constitute part of a cell characteristic in the present invention.

A method according to a seventh aspect is the method according to the sixth aspect, in which the culture data includes one or more of a total number of cells of the target cell, an amount of a cell-secreted substance, an amount of a cell-produced substance, an amount of a cell metabolite, and an amount of a culture medium component, and/or one or more of data on a change in a total number of cells of the target cell over time, a change in an amount of a cell-secreted substance over time, a change in an amount of a cell-produced substance over time, a change in an amount of a cell metabolite over time, and a change in an amount of a culture medium component over time, and in the estimation step, the processor separates the parameters into an input factor for the target cell and an output factor from the target cell, and estimates the cell characteristic such that the input factor and the output factor at a certain time are satisfied. The seventh aspect further specifically defines the estimation of the cell characteristic in the sixth aspect. In the seventh aspect, the phrase "such that an input factor and an output factor are satisfied" means that the cell characteristic is configured such that an output factor obtained as a result of calculation of an input factor on the basis of the cell characteristic is closer to an actual output factor.

A method according to an eighth aspect is the method according to any one of the first to seventh aspects, in which in the prediction step, the processor makes a prediction of the culture result by predicting a biological behavior amount of the target cell, based on the given culture medium composition and a culture condition; changing a cell environment of the target cell, based on a prediction result of the biological behavior amount; and repeating a prediction of the biological behavior amount and a change of the cell environment, based on the changed cell environment. The eighth aspect specifically defines a method for predicting a culture result.

A method according to a ninth aspect is the method according to the eighth aspect, in which the processor performs the prediction of, as the biological behavior amount, a change in a total number of cells over time, a change in a substance produced by the target cell and an amount of the substance over time, and a change in a substance included in a culture environment of the target cell and an amount of the substance over time, and performs the change of, as the cell environment, a total number of cells of the target cell, the substance produced by the target cell, and an amount of the substance. The ninth aspect further specifically defines the prediction according to the eighth aspect.

A method according to a tenth aspect is the method according to any one of the first to ninth aspects, in which in the evaluation step, the processor evaluates a degree of deviation between an estimated value and a true value of the cell characteristic, based on the predicted culture result and the acquired culture result, and in the search determination step, the processor performs a search and determination of the candidate culture medium composition, based on the degree of deviation. The tenth aspect specifically defines a method for evaluating the necessity of further characteristic estimation and for searching for and determining a candidate culture medium composition.

A method according to an eleventh aspect is the method according to any one of the first to ninth aspects, in which in the evaluation step, the processor receives an input of a pair of the culture medium composition and the culture result for the culture medium composition and evaluates a sufficiency of the search, and in the search determination step, the processor performs a search and determination of the candidate culture medium composition, based on an evaluation result of the sufficiency. The eleventh aspect specifically defines another method for evaluating the necessity of further characteristic estimation and for searching for and determining a candidate culture medium composition.

A method according to a twelfth aspect is the method according to any one of the first to eleventh aspects, in which in the control step, the processor determines that the termination criterion is satisfied and terminates the repetition when a difference between the predicted culture result and the acquired culture result and/or a number of times the repetition is performed satisfies a determined condition. The twelfth aspect specifically defines an aspect of a processing termination criterion.

A method according to a thirteenth aspect is the method according to any one of the first to twelfth aspects, in which the target cell is any one of a CHO cell, an HEK cell, or an iPS cell. The thirteenth aspect specifically defines an example of the target cell.

A method according to a fourteenth aspect is the method according to any one of the first to thirteenth aspects, in which the culture result is one or more of an antibody yield or a virus yield by the target cell, a proliferative property of the target cell, and a success rate of inducing differentiation of the target cell into a specific tissue. The fourteenth aspect defines an example of the culture result. In the present invention, it is preferable to use a culture result corresponding to the type of the target cell.

To achieve the object described above, an apparatus according to a fifteenth aspect of the present invention is an apparatus for searching for a culture medium composition for a cell and estimating a cell characteristic. The apparatus includes a processor configured to make a prediction of, for a target cell, a culture result for a given culture medium composition by using a cell characteristic or an estimated value of a cell characteristic of the target cell; perform an acquisition of a culture result of the target cell for all or part of the given culture medium composition; perform an estimation of a cell characteristic of the target cell by using the culture medium composition and the acquired culture result or the predicted culture result; perform an evaluation of necessity to further estimate a cell characteristic of the target cell from the acquired culture result; perform a search and determination of a candidate culture medium composition, based on the necessity and the prediction, the candidate culture medium composition being a culture medium composition for which the culture result is to be acquired next; and perform a repetition of the prediction, the acquisition, the estimation, the evaluation, and the search and determination until a determined termination criterion is satisfied.

According to the fifteenth aspect, as in the first aspect, it is possible to efficiently optimize a culture result even for a cell whose characteristics are unknown. The apparatus according to the fifteenth aspect may include a configuration similar to those according to the second to fourteenth aspects.

To achieve the object described above, a program according to a sixteenth aspect of the present invention is a program for causing a processor to search for a culture medium composition for a cell and estimate a cell characteristic, the program causing the processor to execute a prediction step of making a prediction of, for a target cell, a culture result for a given culture medium composition by using a cell characteristic or an estimated value of a cell characteristic of the target cell; an acquisition step of acquiring a culture result of the target cell for all or part of the given culture medium composition; an estimation step of performing an estimation of a cell characteristic of the target cell by using the culture medium composition and the acquired culture result or the predicted culture result; an evaluation step of performing an evaluation of necessity to further estimate a cell characteristic of the target cell from the acquired culture result; a search determination step of performing a search and determination of a candidate culture medium composition, based on the necessity and the prediction step, the candidate culture medium composition being a culture medium composition for which the culture result is to be acquired next in the acquisition step; and a control step of performing a repetition of the prediction step, the acquisition step, the estimation step, the evaluation step, and the search determination step until a determined termination criterion is satisfied.

According to the sixteenth aspect, as in the first and fifteenth aspects, it is possible to efficiently optimize a culture result even for a cell whose characteristics are unknown. The program according to the sixteenth aspect may include a configuration similar to those according to the second to fourteenth aspects. An aspect of the present invention can also provide a non-transitory tangible recording medium (e.g., various magneto-optical recording devices or semiconductor memories) storing computer-readable code of the program according to these aspects. The term "non-transitory tangible recording medium", as described above, does not include a non-tangible recording medium such as a carrier signal or a propagation signal itself.

As described above, a method, an apparatus, and a program for searching for a culture medium composition and estimating a cell characteristic according to the present invention enable efficient optimization of a culture result even for a cell whose characteristics are unknown.

### BRIEF DESCRIPTION OF THE DRAWINGS

Fig. 1 is a diagram conceptually illustrating a workflow of an entire system;
Fig. 2 is a block diagram illustrating a configuration of a search estimation system according to a first embodiment;
Fig. 3 is a block diagram illustrating a configuration of a processing unit;
Fig. 4 is a diagram conceptually illustrating a configuration of a search estimation system and a culture evaluation system;
Fig. 5 is a flowchart illustrating a process for performing a culture medium composition search and cell characteristic estimation according to the first embodiment;
Fig. 6 is a conceptual diagram for describing evaluation of the necessity of additional estimation of a culture medium composition;
Fig. 7 is a diagram illustrating a phylogenetic tree of CHO cells;
Fig. 8 is a diagram illustrating how prior knowledge is utilized in a search;
Figs. 9A and 9B are diagrams illustrating how a search is performed on the basis of a history;
Fig. 10 is a diagram illustrating an example of a search result for a DG44 line;
Fig. 11 is a diagram illustrating an example of a search result for a K1 line;
Fig. 12 is a diagram illustrating a configuration of a culture result prediction unit;
Fig. 13 is a flowchart illustrating a process for predicting a culture result;
Fig. 14 is a diagram illustrating an overview of a simulator for a biological behavior amount prediction step;
Fig. 15 is a schematic diagram illustrating cellular metabolic pathways;
Fig. 16 is a diagram illustrating an overview of the Michaelis-Menten equation;
Fig. 17 is a diagram illustrating an overview of existing cell metabolism models;
Fig. 18 is a schematic diagram illustrating creation of a cell death model;
Fig. 19 is a diagram illustrating a configuration of a cell characteristic estimation unit;
Fig. 20 is a flowchart illustrating a method for creating a cell mathematical model;
Fig. 21 is a diagram illustrating a feature value extraction step;
Fig. 22 is a flowchart illustrating a mathematical model creation step; and
Fig. 23 is a diagram illustrating the mathematical model creation step.

### DESCRIPTION OF THE PREFERRED EMBODIMENTS

Embodiments of a method, an apparatus, and a program for searching for a culture medium composition and estimating a cell characteristic according to the present invention will be described in detail. In the description, reference is made to the accompanying drawings as necessary.

### Overview of Process

The following describes an example of a search for a composition of a culture medium mainly for antibody-producing CHO cells, with the productivity to be optimized being the antibody yield thereof. However, the target cell in the present invention may be any one of a CHO cell, an HEK cell for producing a virus for gene therapy, and an iPS cell for regenerative medicine, and the intended productivity may also be one or more of the proliferative property of the target cell, the antibody yield or the virus yield of the target cell, and a success rate of inducing differentiation of the target cell into a specific tissue. Even when such target cells and productivities are selected, embodiments and examples having the same gist as those described below can be configured. The culture method can also be selected as appropriate from among well-plate (microtiter plate or microplate) culture, flask culture, batch culture, fed-batch culture, perfusion culture, and the like.

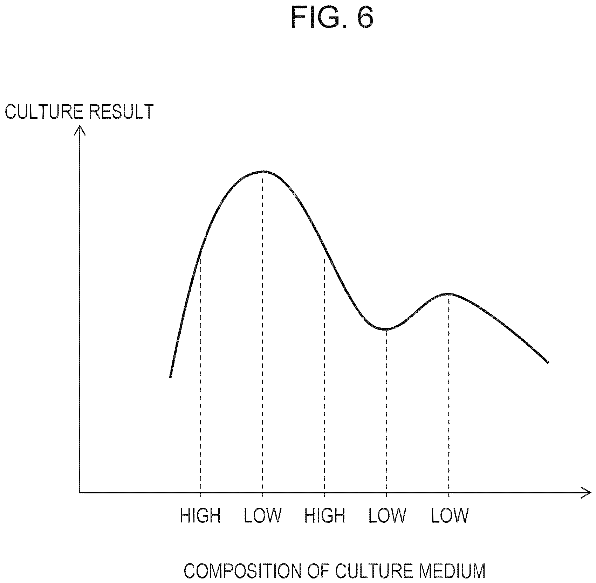

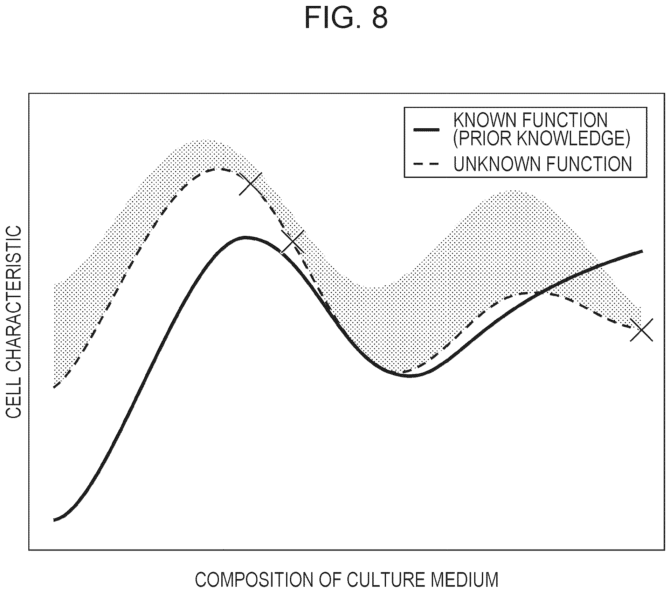

As used herein, the term "culture medium" refers to a mixture of nutrients for culturing cells, and the components thereof typically include, but are not limited to, amino acids, vitamins, metals, and so on. Accordingly, a culture medium can be represented as a multidimensional vector (or a point in a multidimensional space (hereinafter referred to as a "culture medium composition space") having dimensions corresponding to the respective nutrients). The present invention can optimize a partial or entire amount or a content ratio of the composition (nutrients) of such a culture medium. The culture medium composition space is typically multidimensional (the number of dimensions differs depending on the number of nutrients), but may be represented by one dimension, for simplicity, in the following description (such as horizontal axes in Figs. 6 and 8 described below).

### Workflow of the Present Invention

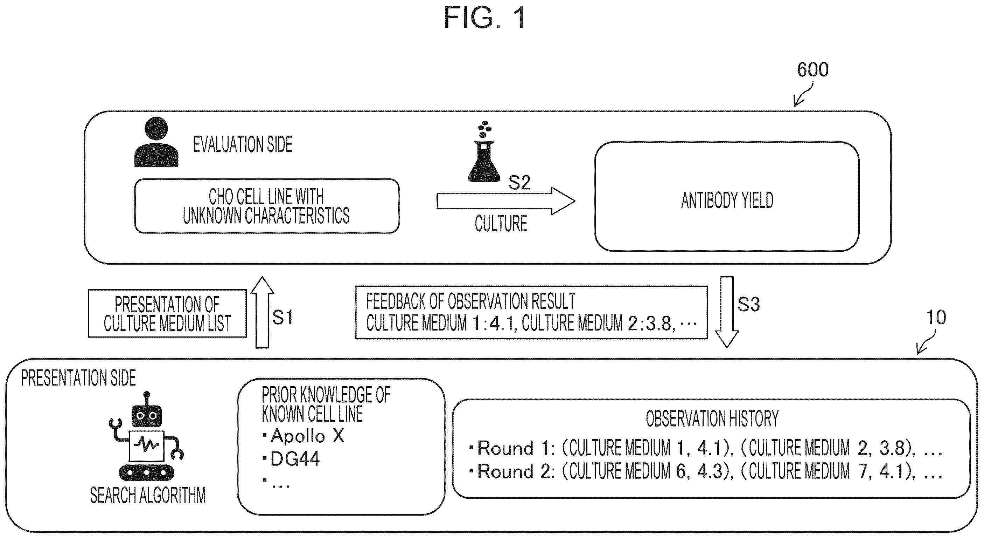

Fig. 1 illustrates a workflow (process overview) assumed in the present invention. A culture medium presentation side (e.g., a search estimation system 10 described below) presents a plurality of candidate culture media (step S1), and an evaluation side (e.g., a user of a culture evaluation system 600 described below) actually cultures a certain cell (target cell) using the candidate culture media to acquire a culture result, and evaluates the result (step S2). The evaluation side (e.g., the culture evaluation system 600 itself) may acquire and evaluate a culture result by simulation or the like without actual culture (step S2). An evaluation result is notified to the presentation side (step S3), and this cycle is repeated.

In such a situation, a purpose of the entire system is to obtain a culture result that is as satisfactory as possible while reducing the number of sets of culture media to be presented and the number of repetitions, or under such constraints. The number of sets of culture media to be presented and the number of repetitions are such that, for example, five culture media are presented and evaluated in steps S 1 to S3 in each round and about three to five rounds are performed, although different conditions may be used. When the round is repeated, the culture media included in the sets to be presented may be changed in accordance with the evaluation results.

In the workflow in Fig. 1, for example, the culture media are CHO cell culture media (CHO: Chinese Hamster Ovary), the term cells are certain CHO cells (including not only original CHO cells but also CHO cells of various lineages caused by mutation, modification, or the like, such as CHO-DG44, CHO-K1, and CHO-S), and the culture result is the antibody yield obtained after culture of the cells for a predetermined period of time. As described above, however, the culture medium, the cells, and the culture result in the present invention are not limited to these examples.

### First Embodiment

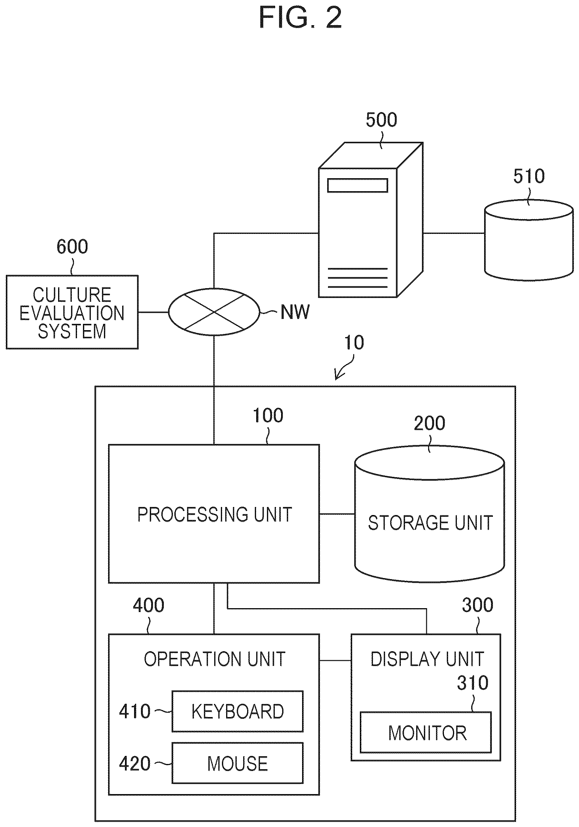

Fig. 2 is a block diagram illustrating a configuration of the search estimation system 10 according to the first embodiment of the present invention and a system related thereto. The search estimation system 10 is a system (search estimation system) for searching for a culture medium composition for a cell and estimating a cell characteristic, and can be implemented using a computer. As illustrated in Fig. 2, the search estimation system 10 includes a processing unit 100 (processor), a storage unit 200, a display unit 300, and an operation unit 400, which are connected to each other to transmit and receive necessary information. These components can be installed in various ways. The components may be installed in one location (such as in one housing or one room) or may be installed in separate locations and connected to each other via a network. The search estimation system 10 can also be connected to an external server 500 and an external database 510 via a network NW, such as the Internet, to acquire information on cell characteristics, culture medium compositions, cell lines, and so on, as necessary.

The culture evaluation system 600 is a system that acquires, evaluates, and manages cell culture data, and is connected to the search estimation system 10 via the network NW. As described below, the search estimation system 10 presents a plurality of candidate culture media, and a user of the culture evaluation system 600 actually cultures a cell (target cell) for the candidate culture media to obtain a culture result of the cell. The culture evaluation system 600 may obtain a culture result of the cell by performing simulation or the like without actual culture. The search estimation system 10 acquires a culture result of the cell for a given culture medium composition from the culture evaluation system 600.

### Configuration of Processing Unit

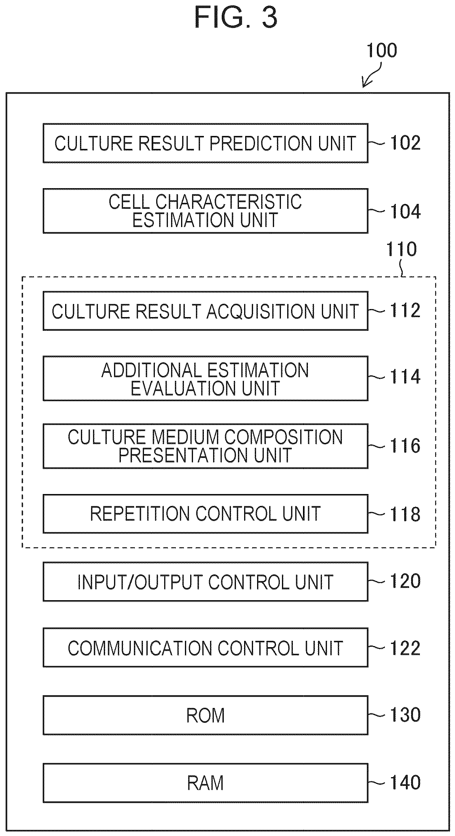

Fig. 3 is a block diagram illustrating a configuration of the processing unit 100 (processor). The processing unit 100 includes a culture result prediction unit 102, a cell characteristic estimation unit 104, a search estimation unit 110, an input/output control unit 120, a communication control unit 122, a ROM 130 (ROM: Read Only Memory), and a RAM 140 (RAM: Random Access Memory). The search estimation unit 110 includes a culture result acquisition unit 112, an additional estimation evaluation unit 114, a culture medium composition presentation unit 116, and a repetition control unit 118. The input/output control unit 120 and the communication control unit 122 control input/output and communication with the external server 500, the external database 510, the culture evaluation system 600, and so on. Search and estimation processing using these components of the processing unit 100 will be described in detail below.

The functions of the components of the processing unit 100 described above can be implemented using various processors. The various processors include, for example, a CPU (Central Processing Unit), which is a general-purpose processor that executes software (program) to implement various functions. The various processors described above also include a programmable logic device (PLD) that is a processor whose circuit configuration can be changed after manufacture, such as an FPGA (Field Programmable Gate Array). The various processors described above further include a dedicated electric circuit that is a processor having a circuit configuration designed specifically for executing specific processing, such as an ASIC (Application Specific Integrated Circuit).

The functions of the components may be implemented by a single processor or a combination of a plurality of processors. Alternatively, a plurality of functions may be implemented by a single processor. Examples of configuring a plurality of functions by a single processor include, first, a form in which, as typified by a computer such as a client or server computer, the single processor is configured by a combination of one or more CPUs and software and the processor is implemented as a plurality of functions. The examples include, second, a form in which, as typified by a system on chip (SoC) or the like, a processor is used in which the functions of the entire system are implemented by a single IC (Integrated Circuit) chip. As described above, the various functions are configured using one or more of the various processors described above as a hardware structure. More specifically, the hardware structure of the various processors is an electric circuit (circuitry) including a combination of circuit elements such as semiconductor elements.

When the processor or electric circuit described above is to execute software (program), the processor (computer) readable code of the software to be executed is stored in a non-transitory tangible recording medium, such as the ROM 130, and the processor refers to the software. The software to be stored in the non-transitory tangible recording medium includes a program (a program for searching for a culture medium composition for a cell and estimating a cell characteristic; hereinafter referred to as a "search estimation program") for executing a method according to the present invention (a method for searching for a culture medium composition for a cell and estimating a cell characteristic; hereinafter referred to as a "search estimation method"). The code may be recorded on a non-transitory tangible recording medium such as various magneto-optical recording devices or a semiconductor memory, instead of the ROM 130. In the processing using software, for example, the RAM 140 is used as a temporary storage area. For example, data stored in an EEPROM (Electronically Erasable and Programmable Read Only Memory) (not illustrated) can be referred to.

The term "non-transitory tangible recording medium", as described above, does not include a non-tangible recording medium such as a carrier signal or a propagation signal itself.

### Configuration of Storage Unit

The storage unit 200 is constituted by a non-transitory tangible recording medium such as various magneto-optical recording media or a semiconductor memory, and an input/output control unit thereof, and stores information such as a culture medium composition, a cell characteristic, and a culture result.

### Configuration of Display Unit and Operation Unit

The display unit 300 includes a monitor 310 (display device) and is capable of displaying input information, information stored in the storage unit 200, a result of processing performed by the processing unit 100, and so on. The operation unit 400 includes a keyboard 410 and a mouse 420 as an input device and/or a pointing device. The user can use these devices and a screen of the monitor 310 to perform an operation necessary to execute the search estimation method and the search estimation program according to the present invention.

### Processing of Search Estimation Method

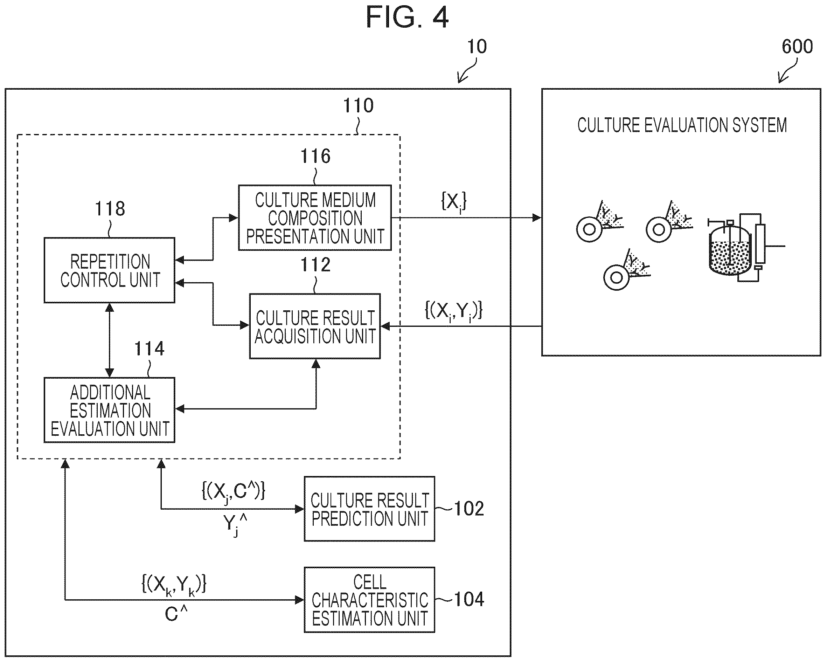

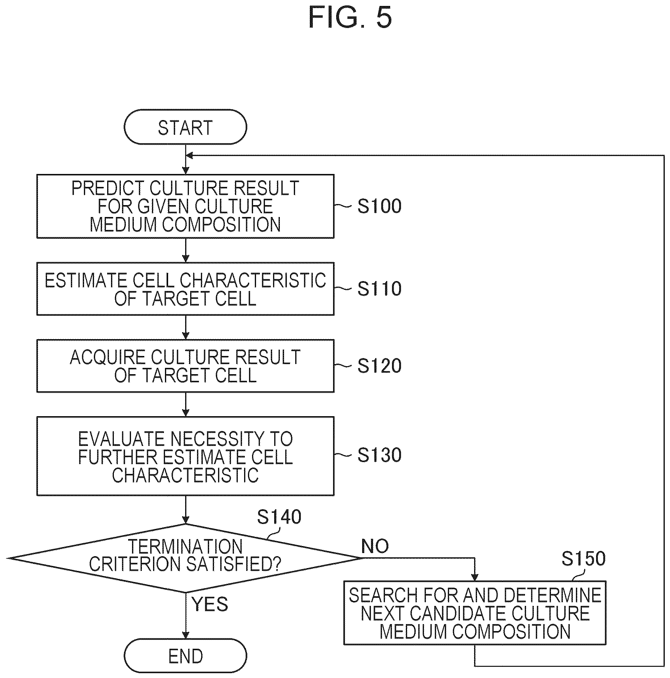

Fig. 4 is a diagram conceptually illustrating a configuration of the search estimation system 10 and the culture evaluation system 600, and Fig. 5 is a flowchart illustrating processing of the search estimation method according to the first embodiment. The processing of the search estimation method and the search estimation program according to the first embodiment will be described hereinafter with reference to these drawings. To simplify the description, the illustration of some components of the search estimation system 10 is omitted, as necessary.

In the first embodiment, a description will be given of an example of a search for a composition of a culture medium for antibody-producing CHO cells, with the productivity to be optimized being the antibody yield thereof.

Here, a culture medium composition is represented by Xᵢ, a cell characteristic is represented by C, and an antibody yield is represented by Yᵢ (the subscript i represents any one of multiple numbers, and it is assumed that the number of cell types is one in a series of workflow steps). Xᵢ can be freely configured, C is unknown, and the true value of Yᵢ is a value that cannot be obtained without experimentation. It is an object to search for Xᵢ that efficiently optimizes (maximizes or utmost increases) Yi, and this object is achieved by constructing a system including a combination of the following elements.

In the first embodiment, Yᵢ to be optimized is the antibody yield, and Yᵢ may be accompanied by other experimental data as appropriate (e.g., other accompanying data such as the number of cells, the time-series change in antibody yield, and changes in culture medium composition may be acquired). The term "experimental data", as used herein, refers to, for example, the number of cells, the time-series change in antibody yield, changes in culture medium composition, or the like, and the cell characteristic estimation unit 104 (the processing unit 100; processor) can use such data for cell characteristic estimation. That is, the cell characteristic estimation unit 104 can estimate a cell characteristic by using not only a culture result but also intermediate experimental data (see "Example of Specific Configuration and Processing of Cell Characteristic Estimation Unit" described below).

### Prediction of Culture Result

The culture result prediction unit 102 (processor) predicts a culture result of the target cell for a given culture medium composition (step S 100: prediction step). The prediction of a culture result can be represented as Yᵢ^ = F(Xi, C). That is, when the culture medium composition Xᵢ and the cell characteristic C are input, the culture result prediction unit 102 predicts an antibody yield Yᵢ^. Here, superscript symbol "^ (hat)" represents a predicted value (the same applies to the following description). However, since the true cell characteristic C is unknown in operation, an estimated value C^ of the cell characteristic is input instead.

In the foregoing description, F may be a function, or may be a predictor obtained by machine learning, a model-based simulator, or the like. When a simulator is used, simple simulation capable of high-speed calculation may be performed. An example of the specific configuration and processing of the culture result prediction unit 102 will be described in detail below (see "Example of Specific Configuration and Processing of Culture Result Prediction Unit"). An experimental system that returns the true value "Yᵢ" described above is also regarded as a type of F (including true C) and is referred to as F0.

### Presentation of Culture Medium Composition

The culture medium composition presentation unit 116 (processor) presents the culture medium composition Xᵢ to the culture evaluation system 600, and the user of the culture evaluation system 600 actually cultures the target cell with the presented culture medium composition Xᵢ to acquire a culture result. Alternatively, the culture evaluation system 600 may acquire the culture result by simulation (e.g., high-accuracy simulation). The culture evaluation system 600 provides the culture result obtained by actual culture or simulation to the search estimation system 10.

### Acquisition of Culture Result

The culture result acquisition unit 112 (processor) acquires a culture result of the target cell for all or part of the given culture medium composition (step S110: acquisition step). The culture result to be acquired is a pair of the culture medium composition Xᵢ and the antibody yield Yᵢ (in Fig. 4, {Xi, Yi}; hereinafter also referred to as "history R").

### Estimation of Cell Characteristic

The cell characteristic estimation unit 104 (processor) estimates a cell characteristic of the target cell by using the culture medium composition and the culture result predicted in step S100 (step S110: estimation step). As used herein, the term "cell characteristic" can be biologically or cytologically defined in various ways. In the present invention, the term "cell characteristic" is defined as "a mechanism for determining the productivity of cells (such as the proliferative property or the antibody yield of the cells, as described above) when a culture medium composition is given". Specifically, the term "cell characteristic" in the present embodiment refers to a mechanism for determining an antibody yield Y for a culture medium composition X, and can be represented by, for example, a function, a machine learning model, or a cell mathematical model. Accordingly, for example, a coefficient of a function, a parameter of machine learning, or a parameter of a metabolic pathway model among cell mathematical models can be considered to be part of a cell characteristic in the present invention.

The estimation of a cell characteristic by the cell characteristic estimation unit 104 in step S110 can be represented as C^ = G({Xᵢ, Yᵢ}). That is, when one or more "pairs of culture medium compositions Xᵢ and antibody yields Yᵢ thereof" (the "history R" described above) are input, the cell characteristic estimation unit 104 estimates a cell characteristic. As described above, Yᵢ may be accompanied by experimental data. Here, G may be a function that returns, for example, one of the functions F, F^ = F_C^(X), in accordance with the cell characteristic described above (it can be regarded as returning C^ in a broad sense). Further, the cell characteristic estimation unit 104 may be a machine-learning parameter determiner, a model-based model estimator, or the like. An example of the specific configuration and processing of the cell characteristic estimation unit 104 will be described in detail below (see "Example of Specific Configuration and Processing of Cell Characteristic Estimation Unit").

When some history R is finally obtained, first, the history R is input to G (the cell characteristic estimation unit 104) to estimate a cell characteristic C^, and then a plurality of candidate culture medium compositions {Xᵢ} are input to F (the culture result prediction unit 102), and a culture medium composition X that returns a maximum antibody yield Y is determined to be an optimum culture medium. However, in view of repetition, it is problematic how to configure a candidate culture medium composition {Xᵢ}, which is a culture medium composition for which a culture result is to be acquired next. Accordingly, in the first embodiment, the following elements are further introduced to the search estimation system 10. Evaluation of Necessity of Additional Estimation

The additional estimation evaluation unit 114 (processor) evaluates the necessity to further estimate a cell characteristic (step S130: evaluation step). The additional estimation evaluation unit 114 can evaluate the necessity of an additional characteristic by using, for example, the following method.

### Evaluation Method 1

Evaluation method 1 can be represented as L = V1({Yᵢ^, Yᵢ}). That is, when a pair of a predicted value Yᵢ^ of the antibody yield (predicted culture result) by the predictor (the culture result prediction unit 102) and a true value Yᵢ of the antibody yield by culture (indicating a difference between the predicted culture result and the acquired culture result; hereinafter also referred to as an "actual difference") is input, the additional estimation evaluation unit 114 evaluates the degree of deviation between the estimated value and the true value of the cell characteristic. If Yᵢ^ is close to Yi, it will be appropriate to determine that C^ is also close to C. Instead of C itself, for example, the degree of deviation between a function F^ incorporating C^ and the true unknown function F0 may be evaluated.

The additional estimation evaluation unit 114 can evaluate that "the necessity of an additional characteristic is high" when the evaluated degree of deviation is large (when a determined degree of deviation is exceeded), and can evaluate that "the necessity of an additional characteristic is low" when the evaluated degree of deviation is small (when it is less than or equal to the determined degree of deviation). If it is evaluated that "the necessity of an additional characteristic is low", the repetition control unit 118 can determine, in step S140, that "a termination criterion is satisfied", and can terminate the repetition.

### Evaluation Method 2

Evaluation method 2 can be represented as L = V2({Xᵢ, Yᵢ}). That is, when the history R (a pair of the culture medium composition Xᵢ and the culture result Yᵢ (e.g., the antibody yield) for the culture medium composition Xᵢ) is input, the additional estimation evaluation unit 114 can evaluate the sufficiency of the search. If {Xᵢ} already fully covers the distribution of possible candidates for the culture medium composition X in the culture medium composition space described above, the additional estimation evaluation unit 114 may evaluate that it is difficult to bring the estimated value and the true value of the cell characteristic closer to each other any further.

In evaluation method 2, furthermore, the additional estimation evaluation unit 114 can further perform evaluation under the assumption that "a change in culture result in response to a change in culture medium composition has continuity". This is because it can be assumed that most cell culture results do not greatly vary with minor changes in culture medium composition. Accordingly, the additional estimation evaluation unit 114 may evaluate that "a region in which the change in Yᵢ is small relative to the change in Xᵢ in the culture medium composition space described above has low necessity of additional estimation" and, conversely, evaluate that "a region in which the change in Yᵢ is large relative to the change in Xᵢ has high necessity of additional estimation". For example, the additional estimation evaluation unit 114 can evaluate that, in Fig. 6 (a conceptual diagram for describing the necessity of additional estimation; the horizontal axis represents a culture medium composition space and the vertical axis represents a culture result), a neighboring region indicated by "high" has "high necessity of additional estimation since the change in Yᵢ is large relative to the change in Xᵢ", and that a neighboring region indicated by "low" has "low necessity of additional estimation since the change in Yi is small relative to the change in Xi".

### Search and Determination of Next Candidate Culture Medium Composition

The repetition control unit 118 (processor) causes the culture medium composition presentation unit 116 (processor) to search for and determine a candidate culture medium composition that is a culture medium composition for which a culture result is to be acquired next (steps S140 and S150: control step and search determination step), on the basis of the necessity of additional estimation, which is evaluated in step S130, and the prediction result in the prediction step.

Specifically, if there is no necessity of additional estimation (YES in step S140: control step), the termination criterion is satisfied. Thus, the repetition control unit 118 (processor) ends the process. If there is necessity of additional estimation, the termination criterion is not satisfied. Thus, the repetition control unit 118 causes the culture medium composition presentation unit 116 (processor) to search for and determine a candidate culture medium composition that is a culture medium composition for which a culture result is to be acquired next (if NO is obtained in step S140, the process proceeds to step S150: control step and search determination step).

In step S140, if the number of repetitions exceeds a predetermined number, the repetition control unit 118 may determine that "the termination criterion is satisfied" and terminate the repetition.

The culture medium composition presentation unit 116 can search for and determine a candidate culture medium composition in the following way in accordance with the evaluation method used by the additional estimation evaluation unit 114. Specifically, when the additional estimation evaluation unit 114 uses evaluation method 1, the culture medium composition presentation unit 116 preferentially sets a candidate culture medium composition X in a range (region) yet to be searched in the culture medium composition space if the degree of deviation is large, and preferentially sets a candidate culture medium composition X for which the culture result Y is predicted to be large from the current estimation result if the degree of deviation is small. When the additional estimation evaluation unit 114 uses evaluation method 2, the culture medium composition presentation unit 116 preferentially sets X in a region where the necessity of additional estimation is high (a region where the change in Yᵢ is large relative the change in Xᵢ), and, conversely, if the necessity of additional estimation is low, the culture medium composition presentation unit 116 preferentially sets X for which Y is predicted to be large from the current estimation result.

### Effect of First Embodiment

As described above, the search estimation system 10 (search estimation method, search estimation system, and search estimation program) according to the first embodiment repeats the prediction and acquisition of a culture result, the estimation of a cell characteristic, the evaluation of the necessity to additionally estimate a cell characteristic, and the search and determination of a candidate culture medium composition, making it possible to efficiently optimize a culture result even for a cell whose characteristics are unknown.

### Additional Embodiment Regarding Cell Characteristic Estimation Method

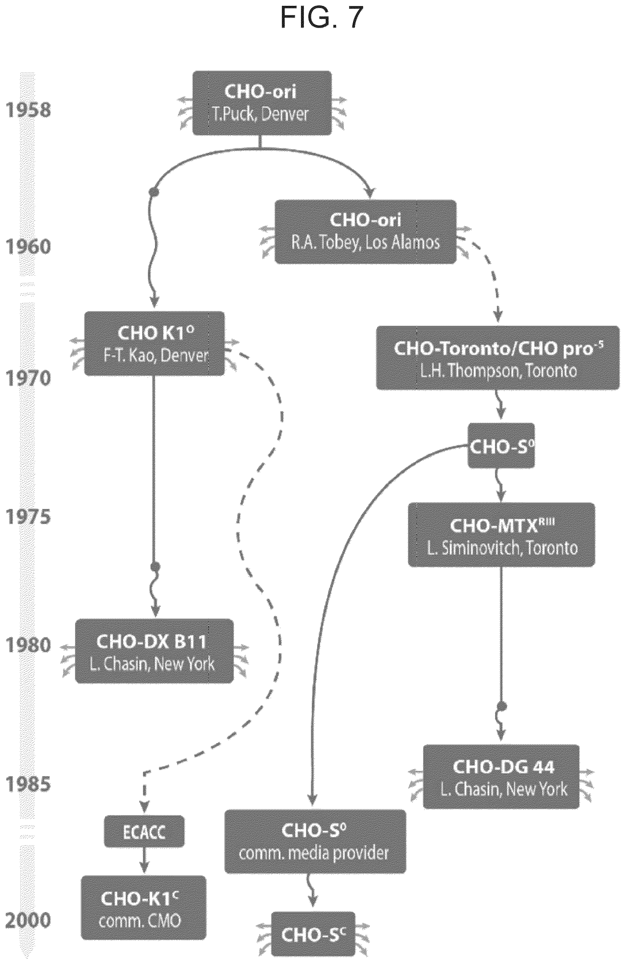

An additional embodiment regarding a cell characteristic estimation method will be described. For example, for CHO cells, it is known that various CHO cell lines are not "equally spaced" from each other, and a relationship like a phylogenetic tree can be schematically represented by a derivation relationship or the like. Specifically, as illustrated in Fig. 7, CHO cells include an original CHO cell (CHO-ori) and a plurality of cell lines having different characteristics from the original CHO cell (CHO-ori), such as "CHO-K1", "CHO-S", and "CHO-DG44".

The phylogenetic tree in Fig. 7 is described in "Wurm, F.M.; Wurm, M.J. Cloning of CHO Cells, Productivity and Genetic Stability A Discussion. Processes 2017, 5, 20.", searched on March 1, 2022 on the Internet (https://www.researchgate.net/publication/316314849_Cloning_of_CHO_Cells_Productivity_ and_Genetic_Stability-A_Discussion/link/59410f0045851566elbeae32/download).

The above article is cited in compliance with http://creativecommons.org/licenses/by/4.0/.

The relationship like a phylogenetic tree described above is not limited to that of CHO cells, and a phylogenetic tree is also present for HEK cells. Such a relationship may also occur when iPS cells are cultured for a long time. The term "cells", as used in the present invention, includes, for example, host cells having certain genes and antibody-producing cells (also referred to as "cell lines") obtained by inserting antibody-producing genes into the host cells, which are generally established by culture, modification, or the like. Such lineages and cell lines may be defined by the genes or may be defined with reference to commonly known lineages (in the case of CHO cells, "CHO-K1", "CHO-S", "CHO-DG44", etc.). The term "cell line" is defined in various ways, and can be defined as, for example, "cells isolated from a living body or cells that are obtained by modifying genes or the like in some way and are capable of long-term stable proliferation and culture while maintaining certain properties".

Based on such cell lineage definition, it is possible to grasp cell characteristics of various available CHO cell lines by some means and configure a set of cell characteristics Cs, such as {C1, C2, C3, ...}, in advance. Accordingly, even a cell with an unknown cell characteristic C belongs to any cell lineage, and thus can be expected to have characteristics close to those of a representative cell of any cell lineage. That is, when a cell characteristic of a CHO cell line is known, it may be assumed that "a true cell characteristic C of an unknown cell will be close to any one of the cell characteristics included in the set Cs" (such an assumption is referred to as "prior knowledge"). In particular, when it is known that an unknown cell is a derivative cell of a specific cell lineage, it may be assumed that C is close to C_k (known information indicating that C belongs to a specific cell lineage is referred to as "prior knowledge").

A text with an underscore, such as "C_k", means that a cell characteristic is set for certain information by using prior knowledge "k". The example described above uses prior knowledge that "a certain unknown cell has a cell characteristic close to a specific cell characteristic Ck among the cell characteristics included in the set Cs".

Then, for example, the initial value of C can be set to C = C_k. Alternatively, F = F_k may be initially set in a system that incorporates C. For example, in Fig. 7, even when it is unknown that a certain cell is CHO-Sc, if it is known that the certain cell is a CHO-S0 derived cell line, it is expected that initially setting "C = C_S0", "F = F_S0", or the like will make the search more efficient than initially setting "C = C_ori" or the like. The subsequent processing can be performed by applying a method similar to that in the first embodiment described above.

As described above, the cell characteristic estimation unit 104 (processor) can perform a search or estimation under the assumption that a cell characteristics of a target cell is similar to a cell characteristic of one or more cells in a known cell group (i.e., by utilizing prior knowledge). Such a search utilizing prior knowledge is illustrated in Fig. 8. In Fig. 8, an unknown function is estimated. A gray shaded region (the width thereof in the vertical axis direction) indicates an estimated width of a cell characteristic for a certain culture medium composition (a point on the horizontal axis). The culture medium composition corresponds to a single point in a culture medium composition space, which is actually a multidimensional space, but is represented by a single point in a one-dimensional space (the horizontal axis in Fig. 8) for convenience in Fig. 8. For cell characteristic estimation, the fact (prior knowledge) that a known function is close to an unknown function is used.

### Aspect in which Solution to Bandit Problem is Applied

Further, a specific embodiment will be described by applying the bandit problem. The bandit problem is a problem of optimizing an unknown function with a limited number of trials on the basis of an observation history. The present invention assigns specific components such as cells, a cell characteristic, and a culture medium composition to such a generalized problem, and further introduces domain-specific elements (such as evaluation of the necessity of additional estimation assuming the continuity of results and the utilization of prior knowledge related to the similarity of a characteristic). Specifically, in this aspect, the culture result is regarded as an observation history, the means for prediction in the prediction step is regarded as an unknown function, and the shape of the unknown function is regarded as a cell characteristic to estimate an unknown function that best fits the observation history.

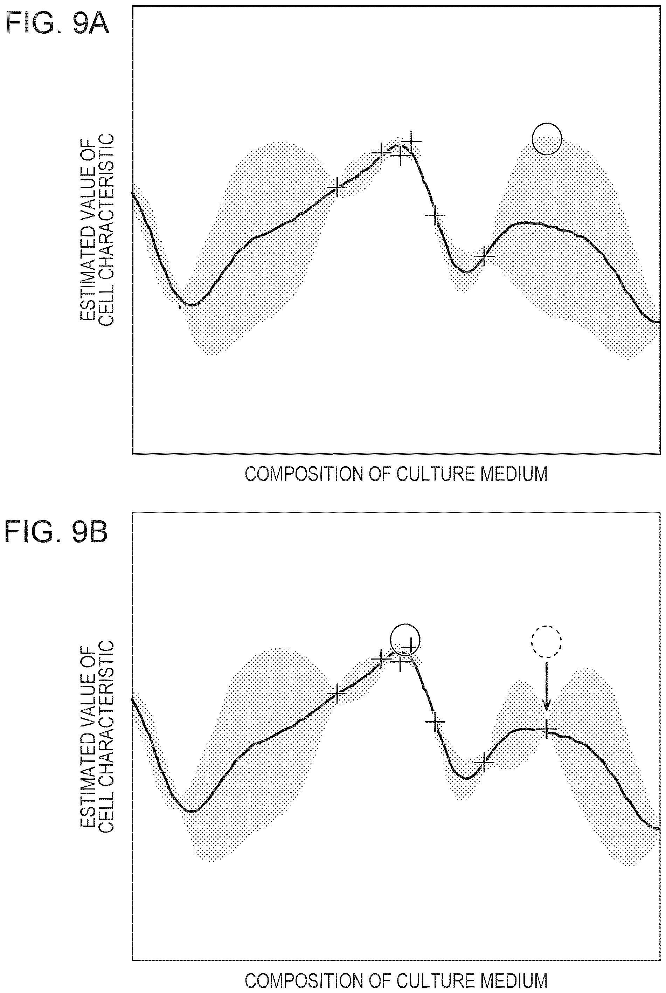

Specifically, for example, GP-UCB (Gaussian Process Upper Confidence Bound) is used to construct Fgp_t(X, C^) = {µ_t(X) + σ_t(x), µ_t(X) - σ_t(X)} (see Figs. 9A and 9B). Note that Fgp is a function constituting the width (interval) of F, µ is a predicted response value (i.e., F^) of F, and σ indicates the uncertainty of prediction. Since F^ is sequentially updated on the basis of the history, Fgp, µ, and σ are different at a time point t of a certain trial. In addition, t(X), σ_t(X), and t(X, C^) are the culture medium composition X, the uncertainty of the cell characteristic C, and the estimated value C^ of the cell characteristic for the culture medium composition X, respectively, in the t-th trial.

Among various culture medium compositions X, a culture medium composition X that maximizes Fupper_t(X_C) = µ_t(X) + σ_t(X) (i.e., the upper limit value of the interval) is one of the candidate culture medium compositions to be presented by the culture medium composition presentation unit 116. The culture medium composition presentation unit 116 can determine, for example, a set of culture medium compositions X that maximize Fupper in a plurality of randomly or appropriately set intervals as a presentation set (candidate culture medium compositions to be presented). This makes it possible to configure the culture medium composition presentation unit 116. In GP-UCB, based on a certain history {Xi, Yi}, F(Xi, C) = µ_t(Xᵢ) = Yᵢ is fixed, and, furthermore, σ_t around Xᵢ is reduced. This makes it possible to configure the cell characteristic estimation unit 104 and the additional estimation evaluation unit 114. That is, Fgp is updated, and the candidate range to be searched is also updated.

Further, for example, when a function for a known cell is represented by F(X, C_k) = µ_k(X), F_0(X, C) = µ_k(X) can be initially set. Thus, the assumption of the similarity of a cell characteristic described above can also be incorporated.

Figs. 9A and 9B illustrate how a search is performed using GP-UCB (a diagram illustrating how prediction is made after a certain number of searches have been performed). In Figs. 9A and 9B, the horizontal axis corresponds to a culture medium composition, and the vertical axis corresponds to an estimated value of a cell characteristic. In Figs. 9A and 9B, a solid line indicates predicted values of the cell characteristic for a certain culture medium composition, and a gray region indicates uncertainty for the predicted values. A cross symbol indicates a previous observation result, and a circle indicates the next search estimation candidate. Fig. 9A illustrates a state in which the culture medium composition corresponding to the circle is determined as a search estimation candidate. The culture medium composition presentation unit 116 selects, as a search estimation candidate, a point at which the cell characteristic is maximum or local maximum. Fig. 9B illustrates a state in which an observation value corresponding to the circle in Fig. 9A is obtained and the uncertainty in the corresponding portion (the portion indicated by a dotted-line circle) has converged. In this state, the culture medium composition presentation unit 116 can further determine the culture medium composition corresponding to the circle (the point at which the cell characteristic is maximum or local maximum) as the next search estimation candidate.

A cell simulator or a similar function thereof may be applied to the culture result prediction unit 102 described above. When a cell simulator is applied, a cell simulation including a metabolic pathway model can be used to express the means for prediction in the prediction step, an unknown function, and/or a similar function for the unknown function (see "Example of Specific Configuration and Processing of Culture Result Prediction Unit" described below).

### EXAMPLE

A search effect of the present invention obtained by simulation is presented. A cell simulator (the culture result prediction unit 102) was configured by a method described below in "Example of Specific Configuration and Processing of Culture Result Prediction Unit". In the following Example, DG44 denotes a DG44 original cell line of CHO cells, X denotes a DG44 derived cell line, and K denotes a K original cell line (for the relationship between these cell lines of the CHO cells, see the lineage diagram in Fig. 7).

In this Example, functions were configured from the simulator in the following cases:
(1) a case where the unknown cell was DG44 (the same lineage as X); and
(2) a case where the unknown cell was K,
and for each of these cases, furthermore;
(a) a case where prior knowledge X was used; and
(b) a case where prior knowledge X was not used.

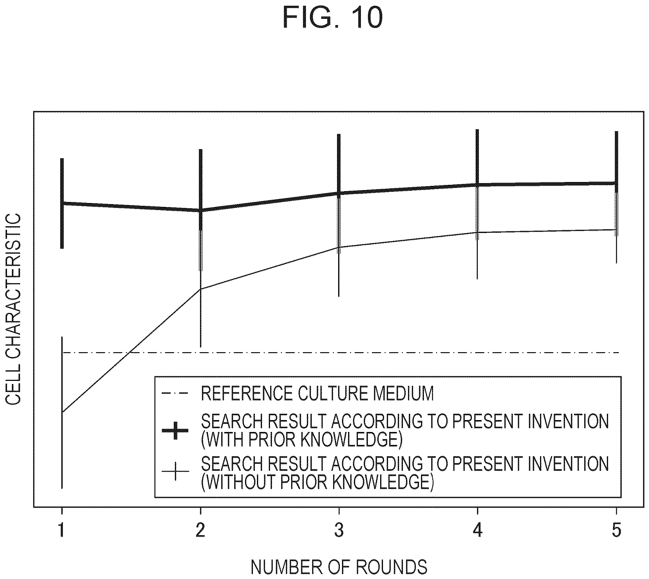

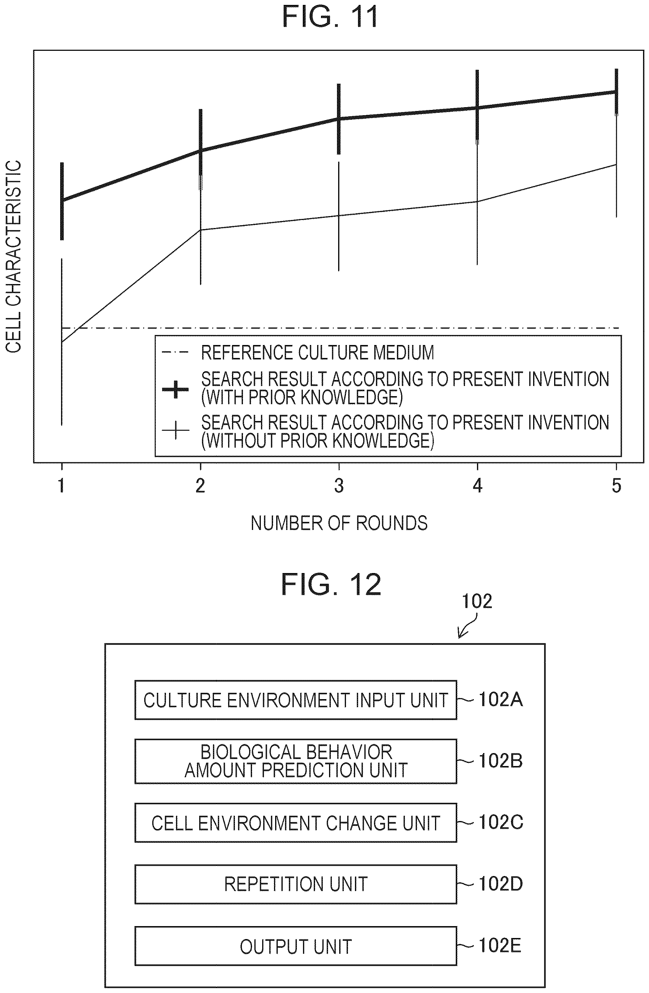

In addition, the effect of the search according to the fourth aspect of the present invention (the aspect of using a solution to the bandit problem) was examined. Then, the number of culture media to be presented at a time was set to five, and it was examined whether a culture medium composition exceeding a reference culture medium could be found in five short trials. The results are illustrated in Figs. 10 and 11. Fig. 10 illustrates a result obtained when the unknown cell is DG44, and Fig. 11 illustrates a result obtained when the unknown cell is K. In Figs. 10 and 11, the horizontal axis represents the number of search trials, and the vertical axis represents the antibody yield (an example of a cell characteristic).

As a result, in both "the case where prior knowledge X was used" and "the case where prior knowledge X was not used", as the number of trials increased, a culture medium composition for which an antibody yield exceeding the reference culture medium was successfully found through limited searches, and it was confirmed that the utilization of prior knowledge was effective particularly for cell lines of the same lineage.

### Example of Specific Configuration and Processing of Culture Result Prediction Unit

An example of a specific configuration and processing of the culture result prediction unit 102 (processor) described above will be described. Fig. 12 is a diagram illustrating an example configuration of the culture result prediction unit 102. In the illustrated example, the culture result prediction unit 102 includes a culture environment input unit 102A, a biological behavior amount prediction unit 102B, a cell environment change unit 102C, a repetition unit 102D, and an output unit 102E. These units can be configured using various processors and memories (such as a ROM and a RAM), as described above for the processing unit 100.

### Method for Predicting Cell Culture Result

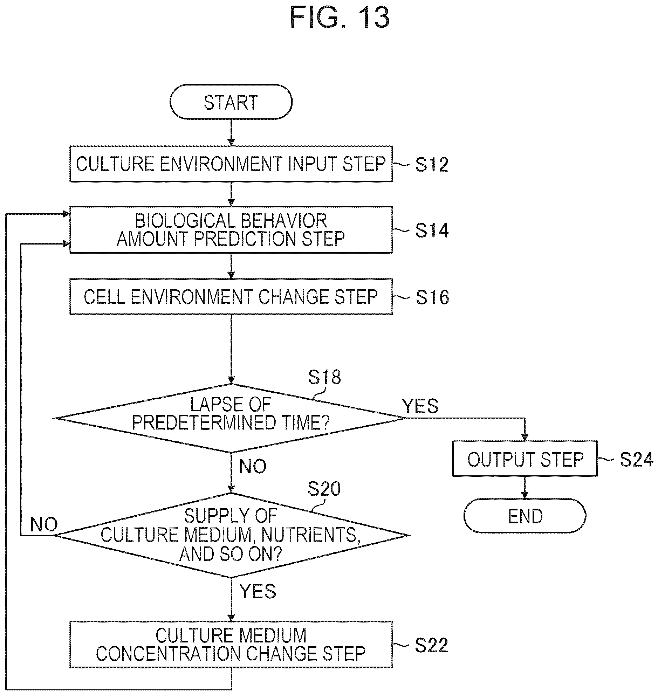

Fig. 13 is a flowchart illustrating a method for predicting a culture result of a target cell, which is performed by the culture result prediction unit 102 having the configuration described above. This method has a culture environment input step of inputting a culture environment constituted by a culture medium composition and culture conditions for culturing cells, a biological behavior amount prediction step of predicting a biological behavior amount on the basis of the culture environment input in the culture environment input step, and a cell environment change step of changing a cell environment on the basis of the biological behavior amount predicted in the biological behavior amount prediction step. The method further has a repetition step of repeating the biological behavior amount prediction step of predicting a biological behavior amount on the basis of the cell environment changed in the cell environment change step and the cell environment change step until a predetermined time that is a culture time elapses. The method further has an output step of outputting the cell environment changed in the cell environment change step after the predetermined time has elapsed.

The steps will be described hereinafter.

### Culture Environment Input Step (Step S 12)

The culture environment input unit 102A performs a culture environment input step (step S12). The culture environment input step is a step of inputting a culture environment constituted by a culture medium composition and culture conditions for culturing cells. The culture environment is input by the user and processed by the culture environment input unit 102A.

Examples of the culture medium composition include culture medium components of a culture medium for culturing the cells and the amounts of the culture medium components. The culture conditions are setting conditions for optimizing a cell culture process. Examples of the culture conditions include conditions such as a culture method, the size and type of a culture vessel, addition of oxygen, supply of a culture medium and a nutrient source, addition of a pH adjuster and carbon dioxide, removal of a culture medium containing growth-inhibiting by-products, and harvest of a target product.

Examples of the culture method include whether to perform sterilization treatment, whether the culture medium is a liquid or a solid, and the culture temperature. The culture conditions are not limited to the conditions at the start of culture, and items during the culture period, such as the presence or absence of addition of oxygen and the amount of addition, the presence or absence of supply of a culture medium and a nutrient source and the amount of supply, addition of a pH adjuster and carbon dioxide, the presence or absence of removal of a culture medium containing growth-inhibiting by-products and the amount of supply, and the presence or absence of harvest of a target product and the amount of harvest can be set as culture conditions.

### Biological Behavior Amount Prediction Step (Step S14)

The biological behavior amount prediction unit 102B performs a biological behavior amount prediction step (step S14). The biological behavior amount prediction step is a step of predicting a biological behavior amount on the basis of the culture environment input in the culture environment input step. The biological behavior amount includes a change in the total number of cells over time, changes in substances produced by the cells and the amounts of the substances over time, and changes in substances included in the culture environment and the amounts of the substances over time. The substances produced by the cells are antibodies and the like produced by the cells. The substances included in the culture environment are culture medium components, by-products, and the like.

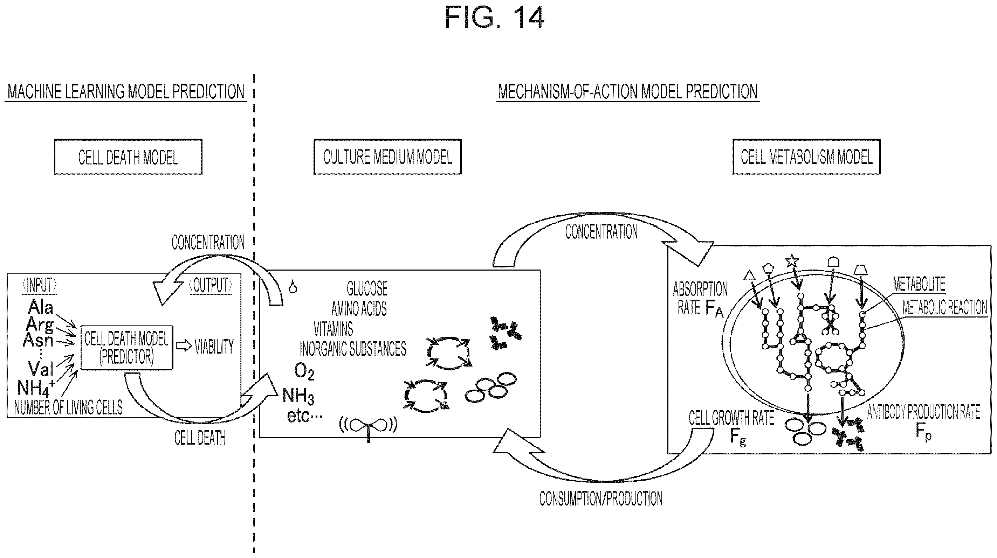

Fig. 14 is a diagram illustrating an overview of a simulator for the biological behavior amount prediction step. As illustrated in Fig. 14, the biological behavior amount prediction step is performed by using a mechanism-of-action model (biological mechanism-of-action model) and a machine learning model. As the mechanism-of-action model, respective mathematical models for the inside and outside of a cell are used. For the inside of the cell, a cell metabolism model for calculating a cell growth rate F_{g} and an antibody production rate Fₚ by using an absorption rate F_{A} at which the cell absorbs each component of the culture medium is used. For the outside of the cell, a culture medium model for calculating a change in the concentration of each component of the culture medium is used. For cell death, a model determined by using machine learning is used.

First, a cell metabolism model performed inside a cell will be described. In the cell metabolism model, the cell growth rate and the antibody production rate can be determined by using a cell culture simulation method that reproduces a bioprocess and the mechanism of a cell to be cultured. The cell culture simulation method can be performed by using a method including a modeling approach including flux balance analysis (FBA) or metabolic flux analysis (MFA) using a genome-scale metabolic model.

The "flux" or "metabolic flux" refers to the rate at which molecules pass through a target pathway or reaction. Among the factors that control flux are the rate of catalysis of enzymes in the pathway, the availability (durability) of substrate, the concentration of enzymes in the cell, and the proximity of enzymes in the pathway. The "metabolic flux analysis method" is a method for determining the amount of molecules that migrate from these factors. The "flux balance analysis" is an analysis method focusing on stoichiometry and metabolic flow rate, and, even when constants related to metabolism are not fully measurable, analyzes the behavior range of a target metabolic circuit and features thereof from the structure of a metabolic reaction on the basis of basic constraint conditions such as the law of conservation of mass.

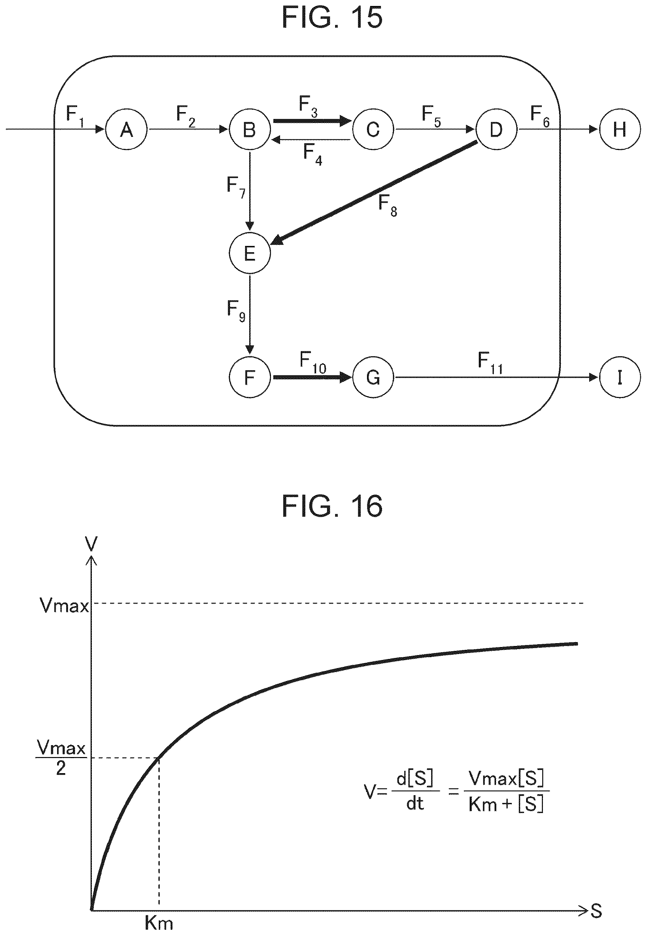

Next, intracellular metabolism will be described with reference to Fig. 15. Fig. 15 is a schematic diagram illustrating an example of cellular metabolic pathways. For a cell metabolism model, first, uptake constraint conditions, which are upper limits and lower limits for uptake of a substrate used for metabolism, nutrients, and the like in a culture medium, are acquired. In Fig. 15, substances generated by metabolism are represented by A, B, C, etc. F₁, F₂, F₃, etc. represent functions indicating the respective changes in the concentrations of the substances over time. For example, F₁ represents a flux at which the substance A is taken in, and F₂ represents a flux at which the substance B is made through metabolism.

In the example illustrated in Fig. 15, the conversion of the substance A into the substance B is performed in equal amounts in accordance with the law of conservation of mass. Likewise, the amount of the substance B is equal to the total amount of conversion into the substance C and the substance E. As described above, the amount of a substance to be converted is determined by the amounts of a substrate and a nutrient to be initially taken in by a cell, and so on. Another parameter that limits the conversion of a substance is the reaction rate. For example, as described above, the conversion of the substance A into the substance B is performed in equal amounts. However, in a certain unit time, the conversion into the substance B has an upper limit due to the limited rate of reaction, and not all the substance A may be converted into the substance B. This parameter for limiting the conversion of a substance is acquired as an uptake constraint condition, and the uptake constraint condition is acquired by using an expression obtained by mathematically modeling a mechanism by which a cell takes in a culture medium component, and is applied to calculation of a culture result.

As a mathematical model for determining the reaction rate, for example, the Michaelis-Menten equation can be used. Fig. 16 illustrates an overview of the Michaelis-Menten equation. The Michaelis-Menten equation is an equation related to a reaction rate V of an enzyme. When a substrate concentration S is low, the reaction rate V is proportional to the substrate concentration S. When the substrate concentration S is high, the reaction rate V converges to a maximum rate Vmax regardless of the substrate concentration S. Other mathematical models that can be used include Fick's law. Fick's law is an expression used to determine a flux that is the amount of substance passing through a unit area per unit time, and the flux is proportional to a diffusion coefficient D and a substrate concentration gradient on both sides of a membrane. In the present embodiment, the Michaelis-Menten equation or Fick's law is used as a constraint condition for taking in a culture medium. This makes it possible to perform a simulation without calculating a condition in which a reaction rate is higher than the reaction rate and the flux determined by the mathematical models but falls within the range of the amount of conversion determined by the law of conservation of mass. Accordingly, more accurate culture prediction can be performed.

The calculation by simulation may be performed by using, in parallel, a plurality of mathematical models that mimic the state of a cell. For example, a mathematical model for cell growth and a mathematical model for production of only bioproduction without cell growth can be arranged in parallel, and the ratio between the plurality of models can be changed in accordance with the culture state such as the culture medium concentration.

Next, a culture medium model performed outside a cell will be described. The culture medium model is a model for determining a change in the concentration of a culture medium surrounding a cell when metabolism occurs under the conditions of the cell metabolism model described above, and a cell signaling model represented by a differential equation can be used. Regarding the concentration change, the Runge-Kutta method can be used to solve an ordinary differential equation for the time t to determine the change in the concentration of each component.

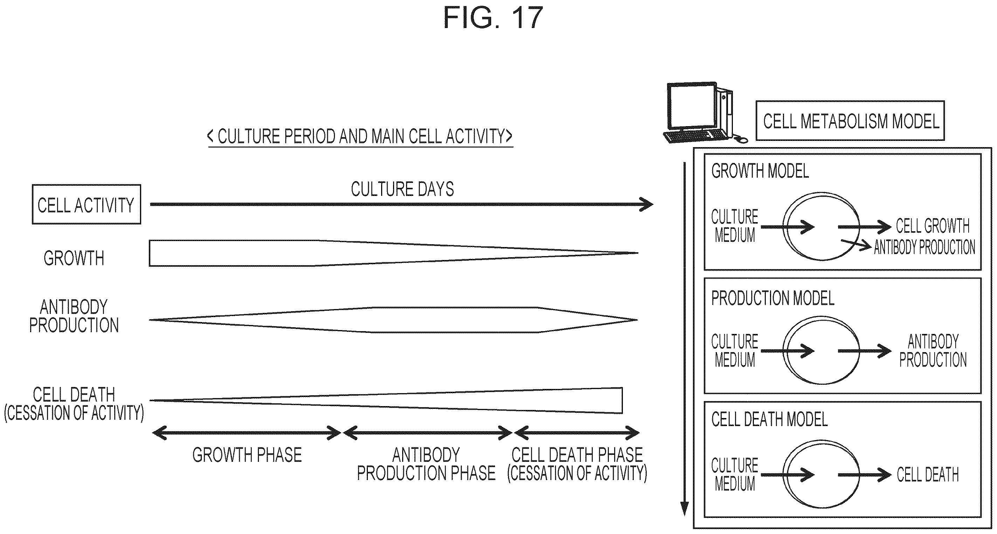

Existing cell metabolism models including a cell death model will now be described. Fig. 17 is a diagram illustrating an overview of existing cell metabolism models. The main cell culture activity during the culture period is divided into cell growth, antibody production by cells, and cell death (cessation of activity). As illustrated in Fig. 17, as the phases of these activities, the early stage of culture has a growth phase in which cells grow, and the middle stage of culture has an antibody production phase in which the cell growth rate decreases and the antibody production rate increases. Subsequently, the late stage of culture has a cell death phase in which cell death, which is the cessation of cellular activity, occurs. In an existing simulation for culture prediction, these three culture activities are performed by using a cell metabolism model, and calculation is performed by using a growth model for cell growth, a production model for antibody production, and a cell death model for cell death. However, the cell death model is implemented as a simple model with constants, and sufficient accuracy is not obtained. In the present embodiment, accordingly, as illustrated in Fig. 14, a trained model obtained by machine learning is used as a cell death model to perform a simulation to calculate a prediction result of culture with high accuracy.

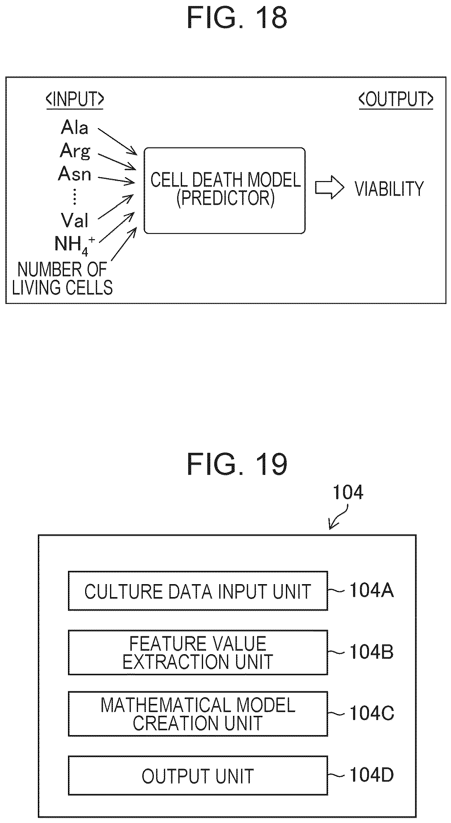

Fig. 18 is a schematic diagram illustrating creation of a cell death model by using machine learning. A random forest method can be used as a method for creating a trained model by machine learning. When a cell death model is constructed by machine learning, the number of living cells, the concentrations of 20 types of amino acids, and the concentration of ammonia are input as data to the input side. The cell viability is input as data to the output side. The data to be used is obtained by actually performing culture, and, for example, 80% of the measured data is used for learning and 20% thereof is used for testing. This makes it possible to create a cell death model.

The creation of a trained model by machine learning is not limited to that for a cell death model. For a data item that is difficult to model based on a mechanism, such as cell death, a trained model is created by machine learning to calculate a prediction result of culture with higher accuracy. Examples of such a data item include growth inhibition and suppression of antibody production. To create a growth inhibition model by machine learning, the amount of cell growth (growth ratio) can be used as data to be used for the output side. To create an antibody production suppression model, the amount of antibody production (production ratio) can be used as data to be used for the output side.

In the biological behavior amount prediction step according to the present embodiment, a trained model created by machine learning is used for cell death, thereby making it possible to measure a biological behavior amount with higher accuracy. As a result, a prediction result of culture can be calculated with high accuracy.

The biological behavior amount prediction step (step S14) can be performed, based on the culture conditions (concentrations of culture medium components) and the uptake constraint conditions acquired in the culture condition input step (step S12), by using a CHO cell simulator including these models (an apparatus or a system that simulates the biological behavior of CHO cells by using a mechanism-of-action model and a trained model created by machine learning for cell death).

### Cell Environment Change Step (Step S16)

The cell environment change unit 102C performs a cell environment change step (step S16). The cell environment change step changes a cell environment on the basis of the biological behavior amount predicted in the biological behavior amount prediction step. The cell environment includes the total number of cells, substances produced by the cells, and the amounts of the substances.

Through the biological behavior amount prediction step, the change in the number of cells over time, the changes in substances produced by the cells and the amounts of the substances over time, and the changes in substances included in the culture environment and the amounts of the substances over time, described above, after a culture time t has elapsed are determined by calculation. In the cell environment change step, the changes in the number of cells and the components of the culture medium after the culture time t has elapsed are reflected in the cell environment.

### Repetition Step

The repetition unit 102D controls implementation of a repetition step. The repetition step is a step of repeating the biological behavior amount prediction step (step S14) and the cell environment change step (step S16).

After the completion of the cell environment change step (step S16), the repetition unit 102D determines whether a predetermined culture time has elapsed (step S18). If the predetermined culture time (e.g., 14 days) for the calculation has elapsed (if YSE is determined), the calculation ends, and the process proceeds to an output step (step S24). If the predetermined culture time has not elapsed (if NO is determined), it is determined whether to supply a culture medium, nutrients, and the like (step S20). If no culture medium, nutrients, or the like is to be supplied (if NO is determined), the process returns to the biological behavior amount prediction step (step S14). If a culture medium, nutrients, and the like are to be supplied (if YES is determined), a culture medium concentration change step (step S22) is performed.

If the supply of a culture medium, nutrients, and the like after a predetermined time has elapsed is set as a culture condition in the culture environment input step (step S12), the culture medium concentration change step (step S22) is performed. The supply of a culture medium, nutrients, and the like also includes the supply of a pH adjuster and carbon dioxide in addition to the culture medium and the nutrients. The culture medium concentration change step adds the supplied culture medium and nutrients to the cell environment at the time point when the cell environment change step is completed to change the culture medium concentration.

The culture medium concentration change step can involve not only supplying a culture medium, nutrients, and the like but also changing the culture medium concentration by removal of a culture medium containing growth-inhibiting by-products and harvest of a target product. If the removal of a culture medium containing growth-inhibiting by-products and the harvest of a target product are set as culture conditions in the culture environment input step (step S12), the removed growth-inhibiting by-products and the harvested target product are removed from the cell environment at the time point when the cell environment change step is completed to change the culture medium concentration.

In the repetition step, an uptake constraint condition is acquired on the basis of the cell environment changed in the cell environment change step (step S16) or the culture medium concentration changed in the culture medium concentration change step (step S22), and the biological behavior amount prediction step (step S14) and the cell environment change step (step S16) are performed. Thereafter, the biological behavior amount prediction step (step S14) and the cell environment change step (step S16) are repeated until the predetermined culture time elapses (step S18).

After the predetermined culture period has elapsed (YES in step S18), the calculation ends. As a result, the number of cells during the culture period and the amount of generation of bioproduction, ammonia, or the like to be generated can be determined. Further, the biological behavior amounts at the respective time points when the repetition step is performed are plotted, thereby making it possible to determine the cell growth curve, the progress of antibody production, and so on.

### Output Step (Step S22)

The output unit 102E performs the output step (step S22). The output step is a step of outputting the cell environment changed in the cell environment change step (step S16).

The output step can output, as results, the number of cells growing during the culture period and the amount of a bioproduction to be generated or the amount of generation of a bioproduction such as an antibody like ammonia. In addition, the cell growth curve, the progress of antibody production, and the like can be output. As the result to be output, the results described above after the lapse of the predetermined time may be output, or the results described above after the lapse of any period during the culture may be output.

### Example of Specific Configuration and Processing of Cell Characteristic Estimation Unit

An example of a specific configuration and processing of the cell characteristic estimation unit 104 (processor) described above will be described. Fig. 19 is a diagram illustrating an example configuration of the cell characteristic estimation unit 104. In the illustrated example, the cell characteristic estimation unit 104 includes a culture data input unit 104A, a feature value extraction unit 104B, a mathematical model creation unit 104C, and an output unit 104D. These units can be configured using various processors and memories (such as a ROM and a RAM), as described above for the processing unit 100.

As described below, the cell characteristic estimation unit 104 can create a cell mathematical model and estimate a cell characteristic by using the created cell mathematical model.

### Method for Creating Cell Mathematical Model

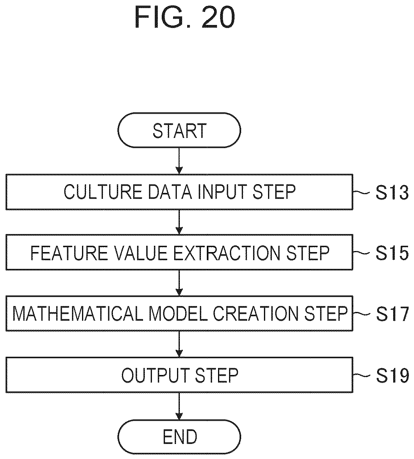

Fig. 20 is a flowchart illustrating a method for creating a cell mathematical model according to the present embodiment. The method according to the present embodiment has a culture data input step (step S13) of inputting culture data of cells, a feature value extraction step (step S15) of extracting feature values of cell activity from the culture data input in the culture data input step, a mathematical model creation step (step S17) of creating a cell mathematical model from the feature values of the cell activity extracted in the feature value extraction step, and an output step (step S19) of outputting the mathematical model created in the mathematical model creation step.

The steps will be described hereinafter.

### Culture Data Input Step (Step S13)

The culture data input unit 104A performs a culture data input step (step S13). The culture data input step is a step of inputting culture data of cells. The culture data can be input in accordance with an input instruction operation performed by the user.

The culture data may include one or more of the total number of cells, the amount of a cell-secreted substance, the amount of a cell-produced substance, the amount of a cell metabolite, and the amount of a culture medium component, and/or one or more of data on a change in the total number of cells over time, a change in the amount of a cell-secreted substance over time, a change in the amount of a cell-produced substance over time, a change in the amount of a cell metabolite over time, and a change in the amount of a culture medium component over time. As used herein, the cell-secreted substance refers to a substance that is not a substance of interest among substances generated by the cells and produced extracellularly. Examples of the cell-secreted substance include ammonia and by-products. The cell-produced substance refers to a substance of interest among substances generated by the cells and produced extracellularly. Examples of the cell-produced substance include antibodies. The cell metabolite refers to a substance present in the cells among substances generated by the cells. As the culture data, culture data obtained by actually performing culture can be used.

### Feature Value Extraction Step (Step S 15)

The feature value extraction unit 104B performs a feature value extraction step (step S15). The feature value extraction step extracts feature values of cell activity from the culture data input in the culture data input step.

The feature values of the cell activity are the concentration of a culture medium component, the consumption amount or consumption rate of the culture medium component, the generation amount or generation rate of the cell-secreted substance, the production amount or production rate of the cell-produced substance, and the generation amount or generation rate of the cell metabolite. In the feature value extraction step, the feature values of the cell activity described above are extracted from the culture data input in the culture data input step.

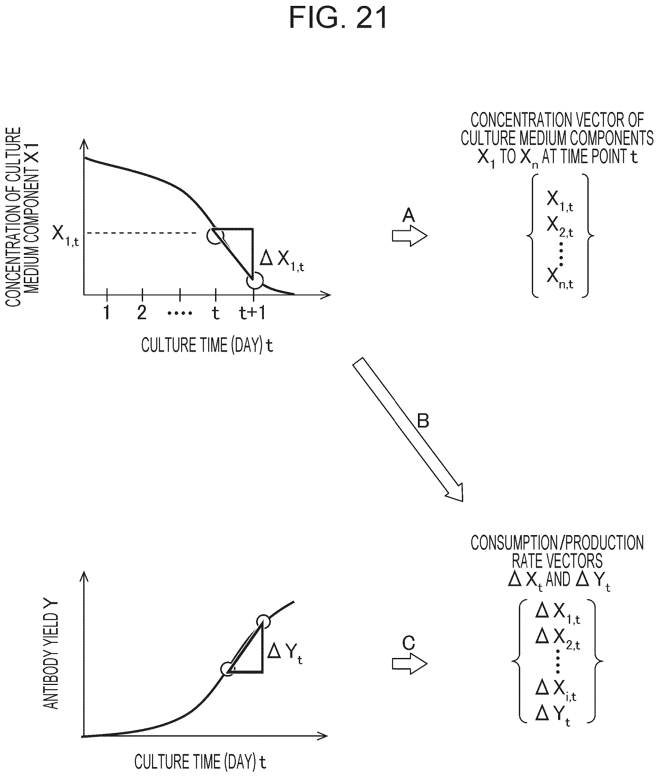

Fig. 21 is a diagram illustrating the feature value extraction step. In Fig. 21, time-series culture data of culture medium component concentrations (for a component X1 as an example) and time-series culture data of the antibody yields (Y) are illustrated. In the feature value extraction step, for example, concentration vectors of culture medium components X1 to Xn at a time point of the culture time t are extracted from the time-series culture data of the culture medium component concentrations illustrated in Fig. 21, as indicated by an arrow A. Further, as indicated by an arrow B, consumption rates ΔX1, t to ΔXn, t of the culture medium components X1 to Xn are extracted. The consumption rate ΔX1, t of the culture medium component X1 is determined by dividing a consumption amount ΔX1 of the culture medium component X1 from time t to time (t + 1) by the culture time to determine the consumption rate ΔX1, t at the time t. The consumption rates ΔX2, t to ΔXn, t of the other culture medium components can also be determined in a similar manner. Likewise, as indicated by an arrow C, a production rate ΔYt of an antibody Y is extracted from the time-series culture data of antibody production. The production rate ΔYt of the antibody Y is determined by dividing the amount of increase ΔY of the antibody Y from the time t to the time (t + 1) by the culture time to determine the production rate ΔYt at the time t.

For the concentration of a culture medium component, preferably, a theoretical upper limit amount of the culture medium component absorbable by cells or a consumption rate at which the cells consume the culture medium component is calculated, and the concentration vector of the culture medium component is extracted. An example of cellular metabolic pathways has been described above with reference to Fig. 15.

In the example of cellular metabolic pathways described above with reference to Fig. 15, the conversion of the substance A into the substance B is performed in equal amounts in accordance with the law of conservation of mass. Likewise, the amount of the substance B is equal to the total amount of conversion into the substance C and the substance E. As described above, the amount of a substance to be converted is determined by the amounts of a substrate and a nutrient to be initially taken in by a cell, and so on. Another parameter that limits the conversion of a substance is the reaction rate. For example, as described above, the conversion of the substance A into the substance B is performed in equal amounts. However, in a certain unit time, the conversion into the substance B has an upper limit due to the limited rate of reaction, and not all the substance A may be converted into the substance B. Accordingly, when the concentration of a culture medium component is high, a difference may occur between the actual concentration of the culture medium component and the concentration of a culture medium component that can be consumed by culture. For this reason, if a cell mathematical model is created by using the actual concentration of the culture medium component, it is feared that the creation of the cell mathematical model to be created will not be based on the cell characteristics. Accordingly, the theoretical upper limit amount of a culture medium that can be absorbed by cells or the consumption rate of the culture medium is calculated and used as a feature value of cell activity. This makes it possible to create a cell mathematical model that is more based on the cell characteristics.

The theoretical upper limit amount that can be absorbed by cells or the consumption rate of the culture medium can be calculated by using, for example, the Michaelis-Menten equation. An overview of the Michaelis-Menten equation is as described above with reference to Fig. 16. The Michaelis-Menten equation is an equation related to the reaction rate V of an enzyme. When the substrate concentration S is low, the reaction rate V is proportional to the substrate concentration S. When the substrate concentration S is high, the reaction rate V converges to the maximum rate Vmax regardless of the substrate concentration S. Other mathematical models that can be used include Fick's law. Fick's law is an expression used to determine a flux that is the amount of substance passing through a unit area per unit time, and the flux is proportional to a diffusion coefficient D and a substrate concentration gradient on both sides of a membrane. The Michaelis-Menten equation or Fick's law is used to calculate the culture medium concentration. This makes it possible to create a cell mathematical model that is more based on the cell characteristics.

### Mathematical Model Creation Step (Step S 17)

The mathematical model creation unit 104C performs a mathematical model creation step (step S17). The mathematical model creation step is a step of creating a cell mathematical model from the feature values of the cell activity extracted in the feature value extraction step.

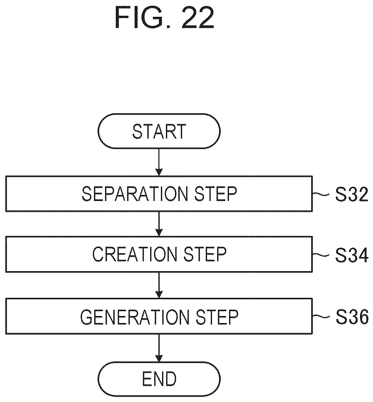

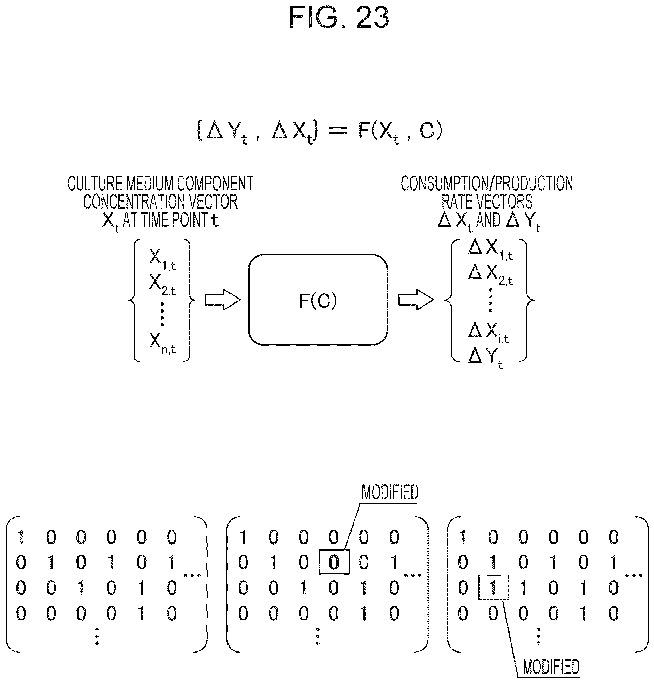

Fig. 22 is a flowchart illustrating the mathematical model creation step. Fig. 23 is a diagram illustrating the mathematical model creation step.

The cell mathematical model creation step separates the feature values of the cell activity extracted in the feature value extraction step into an input factor for the target cell and an output factor from the target cell (separation step: step S32). In the separation step, the concentration of a culture medium component is selected as an input factor. Further, an output factor is selected so as to include at least one of the consumption amount or consumption rate of the culture medium component, the generation amount or generation rate of a cell-secreted substance, the production amount or production rate of a cell-produced substance, or the generation amount or production rate of a cell metabolite.

Then, as illustrated in Fig. 23, F(C) satisfying an input factor and an output factor at any time is searched for to create (search for) a cell mathematical model (creation step: step S34). Here, the model is created by using, as input factors, the concentrations of the culture medium components X1 to Xn at any time point t and using, as output factors, the consumption rates ΔX1, t to ΔXn, t of the culture medium components X1 to Xn at the time point t and the production rate ΔYt of the antibody Y at the time point t. The creation of a cell mathematical model is performed by creating a cell mathematical model that reproduces feature values of cell activity and in which the input factors and the output factors are satisfied, at any time points 11, t2, t3, t4, etc.

Further, the cell mathematical model is based on, as a reference, a cell mathematical model that reproduces the feature values of the cell activity, and the mathematical model creation unit 115 can modify this reference cell mathematical model to generate a plurality of cell mathematical models (generation step: step S36). As illustrated in Fig. 22, the modification of the cell mathematical model can be performed by randomly modifying a reference cell mathematical model. For example, as illustrated in Fig. 23, in a cell mathematical model on the center, the numerical value in the second row and the fourth column is modified from the corresponding value of a reference cell mathematical model (on the left). In a cell mathematical model on the right, the numerical value in the third row and the second column is modified from the corresponding value of the reference cell mathematical model. Further, in random modification, the creation can be performed by using a genetic algorithm. The genetic algorithm is a method for creation through repeated operations such as mutation or crossover. Mutation in the genetic algorithm is an operation of changing a portion of the cell mathematical model. Crossover is a method for selecting one crossover point between two cell mathematical models and swapping portions after the position of the crossover point to create another cell mathematical model.

The mathematical model creation step (step S17) enables creation of a cell mathematical model that reproduces feature values of cell activity. This mathematical model is used as a reference, and a portion of the cell mathematical model is modified to create a cell mathematical model. This makes it possible to easily search for another cell mathematical model that can reproduce culture data even if culture data estimated by using a cell mathematical model that reproduces feature values of cell activity is not a reproduction of the actual culture data.

### Output Step (Step S19)

The output unit 104D performs an output step (step S19). The output step is a step of outputting the cell mathematical model created in the mathematical model creation step (step S17.

In the output step, a plurality of cell mathematical models created in the mathematical model creation step can be output.

The cell characteristic estimation unit 104 (a method for creating a cell mathematical model, a cell mathematical model creation program, and a cell mathematical model creation apparatus) according to the aspect described above can create a cell mathematical model by using culture data obtained by actual culture, and thus can create a cell mathematical model from the culture data without requiring gene-level information.

While an embodiment and other examples of the present invention have been described, the present invention is not limited to the aspects described above and may be modified in various ways.

### Reference Signs List

10 search estimation system
100 processing unit
102 culture result prediction unit
102A culture environment input unit
102B biological behavior amount prediction unit
102C cell environment change unit
102D repetition unit
102E output unit
104 cell characteristic estimation unit
104A culture data input unit
104B feature value extraction unit
104C mathematical model creation unit
104D output unit
110 search estimation unit
112 culture result acquisition unit
114 additional estimation evaluation unit
115 mathematical model creation unit
116 culture medium composition presentation unit
118 control unit
120 input/output control unit
122 communication control unit
130 ROM
140 RAM
200 storage unit
300 display unit
310 monitor
400 operation unit
410 keyboard
420 mouse
500 external server
510 external database
600 culture evaluation system
S1 to S3 workflow step
S12, S14, S16, S18, S20, S22 step of culture result prediction method
S13, S15, S17, S19 step of cell mathematical model creation method
S32, S34, S36 step of mathematical model creation step
S100 to S150 step of search estimation method

## Claims

1. A method for searching for a culture medium composition for a cell and estimating a cell characteristic, the method comprising, by a processor:
a prediction step of making a prediction of, for a target cell, a culture result for a given culture medium composition by using a cell characteristic or an estimated value of a cell characteristic of the target cell;
an acquisition step of acquiring a culture result of the target cell for all or part of the given culture medium composition;
an estimation step of performing an estimation of a cell characteristic of the target cell by using the culture medium composition and the acquired culture result or the predicted culture result;
an evaluation step of performing an evaluation of necessity to further estimate a cell characteristic of the target cell from the acquired culture result;
a search determination step of performing a search and determination of a candidate culture medium composition, based on the necessity and the prediction step, the candidate culture medium composition being a culture medium composition for which the culture result is to be acquired next in the acquisition step; and
a control step of performing a repetition of the prediction step, the acquisition step, the estimation step, the evaluation step, and the search determination step until a determined termination criterion is satisfied.

2. The method according to claim 1, wherein
in the evaluation step, the processor performs the evaluation on an assumption that a change in culture result in response to a change in culture medium composition has continuity.

3. The method according to claim 1 or 2, wherein
in the estimation step, the processor performs the estimation on an assumption that the cell characteristic of the target cell is similar to a cell characteristic of one or more cells in a known cell group.

4. The method according to any one of claims 1 to 3, wherein
when the culture result is regarded as an observation history, a means for the prediction in the prediction step is regarded as an unknown function, and a shape of the unknown function is regarded as the cell characteristic,
the processor uses a solution to a bandit problem for estimating an unknown function that best fits the observation history to perform the prediction step, the estimation step, the acquisition step, the evaluation step, and the search determination step.

5. The method according to claim 4, wherein
the processor uses a cell simulation including a metabolic pathway model to express the means for the prediction in the prediction step, the unknown function, and/or a similar function for the unknown function.

6. The method according to claim 5, wherein
the processor
receives an input of culture data of the target cell,
determines parameters of the metabolic pathway model from the culture data, and
estimates the cell characteristic based on the determined parameters.

7. The method according to claim 6, wherein
the culture data includes one or more of a total number of cells of the target cell, an amount of a cell-secreted substance, an amount of a cell-produced substance, an amount of a cell metabolite, and an amount of a culture medium component, and/or one or more of data on a change in a total number of cells of the target cell over time, a change in an amount of a cell-secreted substance over time, a change in an amount of a cell-produced substance over time, a change in an amount of a cell metabolite over time, and a change in an amount of a culture medium component over time, and
in the estimation step, the processor
separates the parameters into an input factor for the target cell and an output factor from the target cell, and
estimates the cell characteristic such that the input factor and the output factor at a certain time are satisfied.

8. The method according to any one of claims 1 to 7, wherein
in the prediction step, the processor makes a prediction of the culture result by:
predicting a biological behavior amount of the target cell, based on the given culture medium composition and a culture condition;
changing a cell environment of the target cell, based on a prediction result of the biological behavior amount; and
repeating a prediction of the biological behavior amount and a change of the cell environment, based on the changed cell environment.

9. The method according to claim 8, wherein
the processor
performs the prediction of, as the biological behavior amount, a change in a total number of cells over time, a change in a substance produced by the target cell and an amount of the substance over time, and a change in a substance included in a culture environment of the target cell and an amount of the substance over time, and
performs the change of, as the cell environment, a total number of cells of the target cell, the substance produced by the target cell, and an amount of the substance.

10. The method according to any one of claims 1 to 9, wherein
in the evaluation step, the processor evaluates a degree of deviation between an estimated value and a true value of the cell characteristic, based on the predicted culture result and the acquired culture result, and
in the search determination step, the processor performs a search and determination of the candidate culture medium composition, based on the degree of deviation.

11. The method according to any one of claims 1 to 9, wherein
in the evaluation step, the processor receives an input of a pair of the culture medium composition and the culture result for the culture medium composition and evaluates a sufficiency of the search, and
in the search determination step, the processor performs a search and determination of the candidate culture medium composition, based on an evaluation result of the sufficiency.

12. The method according to any one of claims 1 to 11, wherein
in the control step, the processor determines that the termination criterion is satisfied and terminates the repetition when a difference between the predicted culture result and the acquired culture result and/or a number of times the repetition is performed satisfies a determined condition.

13. The method according to any one of claims 1 to 12, wherein
the target cell is any one of a CHO cell, an HEK cell, or an iPS cell.

14. The method according to any one of claims 1 to 13, wherein
the culture result is one or more of an antibody yield or a virus yield by the target cell, a proliferative property of the target cell, and a success rate of inducing differentiation of the target cell into a specific tissue.

15. An apparatus for searching for a culture medium composition for a cell and estimating a cell characteristic, the apparatus comprising a processor configured to:
make a prediction of, for a target cell, a culture result for a given culture medium composition by using a cell characteristic or an estimated value of a cell characteristic of the target cell;
perform an acquisition of a culture result of the target cell for all or part of the given culture medium composition;
perform an estimation of a cell characteristic of the target cell by using the culture medium composition and the acquired culture result or the predicted culture result;
perform an evaluation of necessity to further estimate a cell characteristic of the target cell from the acquired culture result;
perform a search and determination of a candidate culture medium composition, based on the necessity and the prediction, the candidate culture medium composition being a culture medium composition for which the culture result is to be acquired next; and
perform a repetition of the prediction, the acquisition, the estimation, the evaluation, and the search and determination until a determined termination criterion is satisfied.

16. A program for causing a processor to search for a culture medium composition for a cell and estimate a cell characteristic, the program causing the processor to execute:
a prediction step of making a prediction of, for a target cell, a culture result for a given culture medium composition by using a cell characteristic or an estimated value of a cell characteristic of the target cell;
an acquisition step of acquiring a culture result of the target cell for all or part of the given culture medium composition;
an estimation step of performing an estimation of a cell characteristic of the target cell by using the culture medium composition and the acquired culture result or the predicted culture result;
an evaluation step of performing an evaluation of necessity to further estimate a cell characteristic of the target cell from the acquired culture result;
a search determination step of performing a search and determination of a candidate culture medium composition, based on the necessity and the prediction step, the candidate culture medium composition being a culture medium composition for which the culture result is to be acquired next in the acquisition step; and
a control step of performing a repetition of the prediction step, the acquisition step, the estimation step, the evaluation step, and the search determination step until a determined termination criterion is satisfied.

17. A non-transitory computer-readable recording medium storing the program according to claim 16.
